(19) Europäisches Patentamt
European Patent Office
Office européen des brevets

(11) EP 2 571 872 B1

(12) **EUROPEAN PATENT SPECIFICATION**

(45) Date of publication and mention of the grant of the patent:
**31.12.2014 Bulletin 2015/01**

(21) Application number: **11721486.6**

(22) Date of filing: **16.05.2011**

(51) Int Cl.:
***C07D 413/04** (2006.01)* ***C07D 498/04** (2006.01)*
***A61K 31/538** (2006.01)* ***A61K 31/553** (2006.01)*
***A61P 25/28** (2006.01)*

(86) International application number:
**PCT/EP2011/057902**

(87) International publication number:
**WO 2011/144578 (24.11.2011 Gazette 2011/47)**

(54) **COMPOUNDS**

VERBINDUNGEN

COMPOSÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.05.2010 GB 201008209**

(43) Date of publication of application:
**27.03.2013 Bulletin 2013/13**

(73) Proprietor: **Treherne, Johnathan Mark**
**Great Shelford**
**Cambridge, CB22 5LJ (GB)**

(72) Inventors:
- **HORWELL, David Christopher**
**Cambridge**
**Cambridgeshire CB22 6SZ (GB)**
- **SCOPES, David Ian Carter**
**Cambridge**
**Cambridgeshire CB22 3AT (GB)**

(74) Representative: **Chapman, Paul William**
**Kilburn & Strode LLP**
**20 Red Lion Street**
**London**
**WC1R 4PJ (GB)**

(56) References cited:
**EP-A1- 0 055 583**
**WO-A1-2005/080361**
**WO-A1-2009/044160**
**WO-A1-2009/121914**
**WO-A2-2007/079173**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 571 872 B1

## Description

The present invention relates to novel heterocyclic compounds which are useful in the prevention and treatment of neurodegenerative disorders, such as Alzheimer's, Parkinson's and Huntington's diseases as well as type II diabetes.

A number of incurable, ageing-related or degenerative diseases have been linked to a generic and fundamental pathogenic process of protein or peptide misfolding and aggregation. These include Alzheimer's, Parkinson's and Huntington's diseases and type II diabetes. The amyloid deposits present in these diseases consist of particular peptides that are characteristic for each of these diseases but regardless of their sequence the amyloid fibrils have a characteristic β-sheet structure and share a common aggregation pathway. In each disease, a specific protein or peptide misfolds, adopts β-strand structure and oligomerizes to form soluble aggregated intermediates *en route* to fibril formation ultimately forming insoluble amyloid fibres, plaques or inclusions. These insoluble forms of the aggregated protein or peptide form by the intermolecular association of β-strands into β-sheets. Current evidence indicates that the soluble amyloid oligomers may be the principal cause of neurotoxicity (Cleary et al., 2005; Walsh and Selkoe, 2007).

These "amyloid-related" diseases are those in which normally soluble proteins accumulate in various tissues as insoluble deposits of fibrils that are rich in β-sheet structure and have characteristic dye-binding properties (Glenner, 1980a, 1980b). Although the specific polypeptides that comprise the deposits are different for each disease, they have several key features in common. The most prominent of these is the ability of proteins that are highly soluble in biological fluids to be gradually converted into insoluble filamentous polymers enriched in β-sheet conformation. Furthermore, they tend to form by a similar molecular mechanism (by the intermolecular association of β-strands into extended β-sheet), so they tend to share a similar molecular structure and a common ability to bind certain dyes such as Congo Red and Thioflavin T (Selkoe, 2003; Stefani, 2004).

Amyloid-related diseases fall into two main categories: those which affect the brain and other parts of the central nervous system and those which affect other organs or tissues around the body, outside of the brain. Examples of amyloid-related diseases which fall under these two categories are listed below in the following two sections, however many other examples of rare hereditary amyloid-related diseases are known which are not included here and more forms of amyloid-related disease are likely to be discovered in the future.

### Neurodegenerative diseases associated with amyloidosis

Many different neurodegenerative diseases are associated with the misfolding and aggregation of a specific protein or peptide in a particular part of the brain, or elsewhere in the central nervous system, depending on the specific disease (LeVine, 2004; Caughey and Lansbury, 2003; Dev et al., 2003; Taylor et al., 2002; Wood et al., 2003; Masino, 2004; Ross and Poirier, 2004; Soto and Castilla, 2004; Forman et al., 2004). For example:

Various forms of Alzheimer's disease (AD/FAD) as well as Down's syndrome, hereditary cerebral hemorrhage with amyloidosis (HCHWA, Dutch type), cerebral amyloid angiopathy, and possibly also mild cognitive impairment and other forms of dementia are associated with the aggregation of a 40/42-residue peptide called β-amyloid, Aβ(1-40) or Aβ(1-42), which forms insoluble amyloid fibres and plaques in the cerebral cortex, hippocampus or elsewhere in the brain, depending on the specific disease;

Alzheimer's disease is also associated with the formation of neurofibrillary tangles by aggregation of a hyperphosphorylated protein called tau, which also occurs in frontotemporal dementia (Pick's disease);

Parkinson's disease (PD), dementia with Lewy bodies (DLB) and multiple system atrophy (MSA) are associated with the aggregation of a protein called α-synuclein, which results in the formation of insoluble inclusions called "Lewy bodies"; Huntington's disease (HD), spinal and bulbar muscular atrophy (SBMA, also known as Kennedy's disease), dentatorubral pallidoluysian atrophy (DRPLA), different forms of spinocerebellar ataxia (SCA, types 1, 2, 3, 6 and 7), and possibly several other inheritable neurodegenerative diseases are associated with the aggregation of various proteins and peptides that contain abnormally expanded glutamine repeats (extended tracts of polyglutamine);

Creutzfeldt-Jakob disease (CJD), bovine spongiform encephalopathy (BSE) in cows, scrapie in sheep, kuru, Gerstmann-Straussler-Scheinker disease (GSS), fatal familial insomnia, and possibly all other forms of transmissible encephalopathy are associated with the self-propagating misfolding and aggregation of prion proteins;

Amyotrophic lateral sclerosis (ALS), and possibly also some other forms of motor neuron disease (MND) are associated with the aggregation of a protein called superoxide dismutase;

Familial British dementia (FBD) and familial Danish dementia (FDD) are respectively associated with aggregation of the ABri and ADan peptide sequences derived from the BRI protein; and

Hereditary cerebral hemorrhage with amyloidosis (HCHWA, Icelandic type) is associated with the aggregation of a protein called cystatin C.

**Systemic diseases associated with amyloidosis**

In addition to the neurodegenerative diseases listed above, a wide variety of systemic ageing-related or degenerative diseases are associated with the misfolding and aggregation of a particular protein or peptide in various other tissues around the body, outside of the brain (Gejyo et al., 1985; Jaikaran and Clark, 2001; Buxbaum, 2004). For example:

Type II diabetes (also known as adult-onset diabetes, or non-insulin dependent diabetes mellitus) is associated with the aggregation of a 37-residue peptide called the islet amyloid polypeptide (IAPP, or "amylin"), which forms insoluble deposits that are associated with the progressive destruction of insulin-producing β cells in the islets of Langerhans within the pancreas; here also there is evidence that the toxic species are IAPP oligomers (Haataja et al., 2008).
Dialysis-related amyloidosis (DRA) and prostatic amyloid are associated with the aggregation of a protein called $\beta_2$-microglobulin, either in bones, joints and tendons in DRA, which develops during prolonged periods of haemodialysis, or within the prostate in the case of prostatic amyloid;
Primary systemic amyloidosis, systemic AL amyloidosis and myeloma-associated amyloidosis are associated with the aggregation of immunoglobulin light chain (or in some cases immunoglobulin heavy chain) into insoluble amyloid deposits, which gradually accumulate in various major organs such as the liver, kidneys, heart and gastrointestinal (GI) tract;
Reactive systemic AA amyloidosis, secondary systemic amyloidosis, familial Mediterranean fever and chronic inflammatory disease are associated with the aggregation of serum amyloid A protein, which forms insoluble amyloid deposits that accumulate in major organs such as the liver, kidneys and spleen;
Senile systemic amyloidosis (SSA), familial amyloid polyneuropathy (FAP) and familial amyloid cardiomyopathy (FAC) are associated with the misfolding and aggregation of different mutants of transthyretin protein (TTR), which form insoluble inclusions in various organs and tissues such as the heart (especially in FAC), peripheral nerves (especially in FAP) and gastrointestinal (GI) tract;
Another form of familial amyloid polyneuropathy (FAP, type II) is associated with the aggregation of apolipoprotein AI in the peripheral nerves;
Familial visceral amyloidosis and hereditary non-neuropathic systemic amyloidosis are associated with misfolding and aggregation of various mutants of lysozyme, which form insoluble deposits in major organs such as the liver, kidneys and spleen;
Finnish hereditary systemic amyloidosis is associated with aggregation of a protein called gelsolin in the eyes (particularly in the cornea);
Fibrinogen α-chain amyloidosis is associated with aggregation of the fibrinogen A α-chain, which forms insoluble amyloid deposits in various organs such as the liver and kidneys;
Insulin-related amyloidosis occurs by the aggregation of insulin at the site of injection in diabetics;
Medullary carcinoma of the thyroid is associated with the aggregation of calcitonin in surrounding tissues;
Isolated atrial amyloidosis is associated with the aggregation of atrial natriuretic peptide (ANP) in the heart; and
Various forms of cataract are associated with the aggregation of y-crystallin proteins in the lens of the eyes.

**Pathogenic mechanism of amyloid-related diseases**

While all these amyloid-related diseases share a common association with the pathogenic process of amyloidosis, the precise molecular mechanism by which this generic process of protein/peptide misfolding and aggregation is linked to the progressive degeneration of affected tissues is unclear. In some cases, including many of the systemic amyloid-related diseases, it is thought that the sheer mass of insoluble protein or peptide simply overwhelms the affected tissues, ultimately leading to acute organ failure. In other cases, including most of the neurodegenerative diseases listed above, it is increasingly appreciated that the toxic forms of amyloidogenic proteins are soluble oligomeric species which range in size from dimers and trimers, to much larger species comprising tens or even hundreds or thousands of protein or peptide monomers. Moreover, the oligomers are inherently toxic to cells *in vitro* in the absence of insoluble aggregates, and they appear to share a common structural feature as they can all be recognised by the same antibody despite the fact that they may be formed by proteins or peptides with very different amino acid sequences (Kayed et al., 2003; Glabe, 2004; Walsh et al., 2002; Walsh and Selkoe, 2004). In the case of Alzheimer's disease several studies have shown that disease severity correlates more closely with soluble forms of Aβ than with fibrillar forms of the peptide (Lue et al., 1999; McLean et al., 1999; Wang et al., 1999) and points to a key pathogenic role for soluble oligomers of Aβ.
Accumulating evidence (Estrada and Soto, 2007) provides a cogent argument that compounds which block Aβ aggregation and prevent the generation of toxic Aβ assemblies may provide successful new treatments for AD,. Unlike current therapies (Melnikova, 2007; Shah et al., 2008; Wilcock, 2008) which only treat the symptoms of the disease, such 'disease-modifying' therapies may have the potential to slow or even halt disease progression. Molecules that inhibit Aβ aggregation and prevent oligomerization or are capable of binding to toxic Aβ assemblies and neutralize their

toxic effects may be useful in the treatment of AD. Furthermore, preventing Aβ aggregation is therapeutically attractive because this process is believed to be an exclusively pathological event and compounds targeting this mechanism are more likely to have a better safety profile compared to some other approaches currently being pursued.

The molecular structure of these toxic soluble oligomers is not known and the precise mechanism by which they kill cells is also unclear, but several theories have been proposed. According to just one theory called the "channel hypothesis", for example, the oligomers form heterogeneous pores or leaky ion channels, which allow ions to flow freely through cell membranes, thereby destroying their integrity which ultimately causes cell death (Kagan et al., 2002).

Regardless of the precise pathogenic mechanism, however, an overwhelming amount of evidence has now been accumulated which suggests that the general process of protein/peptide aggregation is the primary cause of all these, and possibly other, different amyloid-related diseases.

WO 2009/044160 discloses pyridine derivatives useful for treating amyloid-related diseases, e.g. Alzheimer's disease.

WO 2007/079173 discloses certain 2-heteroaryloxy - phenol derivatives as antibacterial agents.

WO 2005/080361 discloses certain cyclobutyl aryl diamines as histamine-3 receptor modulators.

EP-0055583 discloses novel derivatives of 4-amino-6, 7-dimethoxy-2-piperazinoquinazoline for use in treating hypertension.

WO2009/121914 discloses certain benzoxazine derivatives for the treatment of Alzheimer's disease.

The present invention relates to chemical compounds and compositions which are inhibitors of amyloid toxicity and as such have use in the treatment of amyloid-related diseases and disorders.

Thus, in a first aspect, the present invention provides a compound of formula (I) or a pharmaceutically acceptable salt thereof:

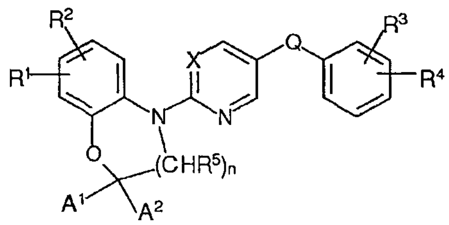

(I)

wherein

X is N or CH;

Q is $NR^6$ or O;

$A^1$ and $A^2$ are independently hydrogen or $C_{1-6}$ alkyl or may together form a carbonyl group;

$R^1$ and $R^2$ are independently hydrogen, halogen, $CF_3$, CN, $OR^7$, $OR^8$, $NR^8R^9$, $NR^8COR^{10}$, $NR^8SO_2R^{10}$, $SO_2NR^8R^9$, $SO_2R^{10}$ or $C_{1-6}$ alkyl optionally and independently substituted by one or more of hydroxyl, $C_{1-6}$ alkoxy, halogen or $NR^8R^9$;

$R^3$ is hydrogen, halogen, $CF_3$ or $OR^7$;

$R^4$ is hydrogen, halogen, $CF_3$, $OR^8$, $NR^8R^9$, $NR^8COR^{10}$, $NR^8SO_2R^{10}$ or $C_{1-6}$ alkyl optionally substituted by hydroxyl, $C_{1-6}$ alkoxy or $NR^8R^9$;

or when $R^3$ and $R^4$ are positioned ortho and taken together form $-O(CH_2)_mO-$, where m is 1-3;

$R^5$ is hydrogen or $C_{1-6}$ alkyl optionally substituted by hydroxyl, $C_{1-6}$ alkoxy or $NR^8R^9$;

$R^6$ is hydrogen or $C_{1-6}$ alkyl;

$R^7$ is hydrogen or $C_{1-6}$ alkyl optionally substituted by $OR^8$ or $NR^8R^9$;

$R^8$ is hydrogen, $C_{1-6}$ alkyl, optionally substituted by hydroxyl or $C_{1-6}$ alkoxy or $C_{1-3}$ alkylphenyl wherein said phenyl

group is optionally substituted by one or more substituents selected from halogen, $C_{1-6}$ alkyl, $CF_3$, $OR^7$, $NR^8R^9$ or $OCF_3$;

$R^9$ is hydrogen, $C_{1-6}$ alkyl, $C_{1-6}$ alkenyl, $C_{1-6}$ alkynyl, phenyl or $C_{1-3}$ alkylphenyl wherein said phenyl groups are optionally substituted by one or more substituents selected from halogen, $C_{1-6}$ alkyl, $CF_3$, $OR^7$ or $OCF_3$;

or the groups $R^8$ and $R^9$ when they are attached to a nitrogen atom may together form a 5- or 6-membered ring which optionally contains one further heteroatom selected from $NR^7$, S and O said 5 or 6 membered ring being optionally substituted by hydroxyl or $C_{1-6}$ alkoxy;

or the groups $R^8$ and $R^9$ when they are attached to a nitrogen atom may together form an azetidinyl ring optionally substituted by hydroxyl or $C_{1-6}$ alkoxy; and

$R^{10}$ is $C_{1-6}$ alkyl or a phenyl group optionally substituted by one or more substituents selected from halogen, $C_{1-6}$ alkyl, $CF_3$, $OCF_3$ or $OR^7$.

n is 1 or 2;

As used herein "postitioned ortho" means that $R_3$ and $R_4$ are on adjacent carbon atoms. They can be taken together to form $-O(CH_2)_mO-$, where m is 1-3. m is preferably 1 or 2. Examples of such groups include $-OCH_2O-$, $-OCH_2CH_2O$ or $-OCH_2CH_2CH_2O-$. These groups together with the carbon atoms to which they are attached form a 5-, 6-or 7- membered ring.

The term "alkyl" as used herein whether on its own or as part of a larger group e.g. "alkoxy" or "alkylphenyl" includes both straight and branched chain radicals, including but not limited to methyl, ethyl, n-propyl, isopropyl, n-butyl, sec-butyl and tert-butyl. The term alkyl also includes those radicals wherein one or more hydrogen atoms are replaced by fluorine, e.g. $CF_3$.

The term "alkenyl" and "alkynyl" as used herein includes both straight and branched chain radicals.

The term "halogen" as used herein includes fluorine, chlorine and bromine

Preferred compounds are:

8-Fluoro-4-[5-(3-morpholinophenoxy)pyrimidin-2-yl]-2,3-dihydro-1,4-benzoxazine

6-Fluoro-4-[5-(3-morpholinophenoxy)pyrimidin-2-yl]-2,3-dihydro-1,4-benzoxazine

6-Cyano-4-[5-(3-morpholinophenoxy)pyrimidin-2-yl]-2,3-dihydro-1,4-benzoxazine

4-[5-(3-morpholinophenoxy)pyrimidin-2-yl]-2,3-dihydro-1,4-benzoxazine

8-Fluoro-4-[5-(3-morpholinophenoxy)-2-pyridyl]-2,3-dihydro-1,4-benzoxazine

9-Fluoro-5-[5-(3-morpholin-4-ylphenoxy)pyrimidin-2-yl]-2,3,4,5-tetrahydro-benzo-[b][1,4]oxazepine

1-[5-(3-Morpholin-4-ylphenoxy)pyrimidin-2-yl]-2,3-dihydro-1H-pyrido-[2,3-b][1,4]-oxazine

(S)-8-Fluoro-4-[5-(3-methylmorpholin-4-ylphenoxy)pyrimidin-2-yl]-2,3-dihydro-1,4-benzoxazine

(R)-8-Fluoro-4-[5-(3-methylmorpholin-4-ylphenoxy)pyrimidin-2-yl]-2,3-dihydro-1,4-benzoxazine

8-Fluoro-4-[5-(3-methoxyphenoxy)pyrimidin-2-yl]-2,3-dihydro-1,4-benzoxazine 8-Fluoro-4-[5-(3-N,N-dimethylaminophenoxy)pyrimidin-2-yl]-2,3-dihydro-1,4-benzoxazine

4-((5-(3-bromophenoxy)pyrimidine-2yl)-8-fluoro-3,4-dihydro-2H-benzo[1,4]oxazine 1-{3-[2-(8-Fluoro-2,3-dihydrobenzo[1,4]oxazin-4-yl)-pyrimidin-5-yloxy]-phenyl}-azetidin-3-ol

4-[5-(3-Morpholin-4-yl-phenoxy)pyridin-2-yl]-3,4-dihydro-2H-benzo[1,4]oxazine-6-carbonitrile

8-Fluoro-3-methyl-4-[5-(3-morpholin-4-yl-phenoxy)pyrimidin-2-yl]-3,4-dihydro-2H-benzo [1,4] oxazine

The present invention encompasses any salt of a compound of the present invention and, in particular, any pharmacologically acceptable salt. Preferred salts for the present invention include hydrohalogenates (for instance, hydrochloride salt, hydrobromide salt, hydroiodide salt and the like), inorganic acid salts (for instance, sulphate salt, nitrate salt, perchlorate salt, phosphate salt, carbonate salt, bicarbonate salt and the like), organic carboxylic acid salts (for instance, acetate salt, maleate salt, tartrate salt, fumarate salt, citrate salt and the like), organic sulfonic acid salts (for instance, methanesulfonate salt, ethane sulfonate salt, benzenesulfonate salt, toluenesulfonate salt, camphorsulfonate salt and the like), amino acid salt (for instance, aspartate salt, glutamate salt and the like), quaternary ammonium salts, alkaline metal salts (for instance, sodium salt, potassium salt and the like), alkaline earth metal salts (for instance, magnesium salt, calcium salt and the like) and the like.

Salts may be prepared in a conventional manner using methods well known in the art. Acid addition salts of said basic compounds may be prepared by dissolving the free base compounds according to the first aspect of the invention in aqueous or aqueous alcohol solution or other suitable solvents containing the required acid. Where a compound of the invention contains an acidic function, a base salt of said compound may be prepared by reacting said compound with a suitable base. The acid or base salt may separate directly or can be obtained by concentrating the solution e.g. by evaporation.

The compounds of the invention may exist in the form of optical isomers, e.g. diastereoisomers and mixtures of isomers in all ratios, e.g. racemic mixtures. The invention includes in particular the isomeric forms (R or S). The different isomeric forms may be separated or resolved one from the other by conventional methods, or any given isomer may be obtained by conventional synthetic methods or by stereospecific or asymmetric synthesis. Where a compound contains an alkene moiety, the alkene can be presented as a *cis* or *trans* isomer or a mixture thereof. When an isomeric form of a compound of the invention is provided substantially free of other isomers, it will preferably contain less than 5% w/w, more preferably less than 2% w/w and especially less than 1% w/w of the other isomers.

Since the compounds of the invention are intended for use in pharmaceutical compositions, it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions; these less pure preparations of the compounds should contain at least 1%, more suitably at least 5%, e.g. 10 to 59% of a compound of the formula (I).

A compound of formula (I), wherein $R^1$, $R^2$, $R^3$, $R^4$, $R^5$, $A^1$ and $A^2$ are as defined for formula (I), X is a nitrogen atom and Q is an oxygen atom may be prepared from a compound of formula (II)

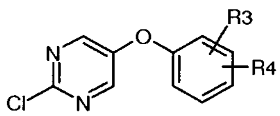

(II)

wherein $R^3$ and $R^4$ are as defined in formula (I), by treatment with an appropriate cyclic aniline in the presence of a suitable catalyst such as *tris*(dibenzylideneacetone)-palladium(0), a phosphine ligand such as 4,5-*bis*(diphenylphosphino)-9,9-dimethylxanthene and a base such as cesium carbonate in a solvent such as 1,4-dioxan with heating.

A compound of formula (II) wherein $R^3$ and $R^4$ are as defined in formula (I), may be prepared by treatment of 2-chloro-5-hydroxypyrimidine with one equivalent of an appropriate boronic acid of formula (III), wherein $R^3$ and $R^4$ are as defined in formula (I), in a suitable solvent such as dichloromethane in the presence of triethylamine and copper(II) acetate. 2-Chloro-5-hydroxypyrimidine may be prepared by methods well known to those skilled in the art.

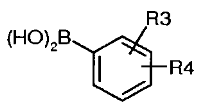

(III)

A compound of formula (I), wherein $R^1$, $R^2$, $R^3$, $R^4$, $R^5$, $A^1$ and $A^2$ are as defined for formula (I),X is a carbon atom and Q is an oxygen atom may be prepared from a compound of formula (IV)

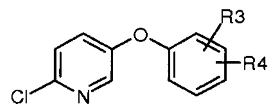

(IV)

wherein $R^3$ and $R^4$ are as defined in formula (I), by treatment with an appropriate cyclic aniline in the presence of a suitable catalyst such as *tris*(dibenzylideneacetone)-palladium(0), a phosphine ligand such as 4,5-*bis*(diphenylphosphino)-9,9-dimethylxanthene and a base such as cesium carbonate in a solvent such as 1,4-dioxan with heating.

A compound of formula (IV) wherein $R^3$ and $R^4$ are as defined in formula (I), may be prepared by treatment of 2-chloro-5-hydroxypyridine with one equivalent of an appropriate boronic acid of formula (III), wherein $R^3$ and $R^4$ are as defined in formula (I), in a suitable solvent such as dichloromethane in the presence of triethylamine and copper(II) acetate. 2-Chloro-5-hydroxypyridine is commercially available.

Alternatively, a compound of formula (I), wherein $R^1$, $R^2$, $R^3$, $R^4$, $R^5$, $A^1$ and $A^2$ are as defined for formula (I), X is a nitrogen atom and Q is an oxygen atom, may be prepared from a compound of formula (V) by treatment with a boronic acid of formula (III) wherein $R^3$ and $R^4$ are as defined in formula (I), in a suitable solvent such as dichloromethane in the presence of triethylamine and copper(II) acetate.

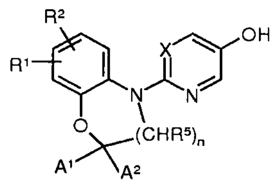

(V)

A compound of formula (V) can be prepared from a compound of formula (VI) by debenzylation using classical hydrogenolysis conditions of hydrogen gas in the presence of a suitable catalyst such as palladium on carbon in a suitable solvent such as ethanol.

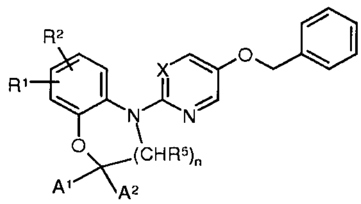

(VI)

A compound of formula (VI) can be prepared from 2-chloro-5-benzyloxypyrimidine using an appropriate aniline in the presence of a suitable catalyst such as *tris*(dibenzylideneacetone)-palladium(0), a phosphine ligand such as 4,5-*bis*(diphenylphosphino)-9,9-dimethylxanthene and a base such as cesium carbonate in a solvent such as 1,4-dioxan with heating.

A compound of formula (I), wherein $R^1$, $R^2$, $R^3$, $R^4$, $R^5$, $A^1$ and $A^2$ are as defined for formula (I), X and Q are nitrogen atoms, may be prepared from a compound of formula (VII)

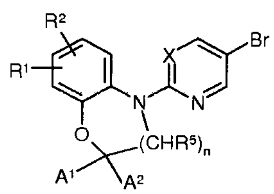

(VII)

wherein $R^1$, $R^2$, $R^5$, $A^1$ and $A^2$ are as defined in formula (I) by treatment with an appropriate aniline in the presence of a suitable catalyst such as *tris*-(dibenzylideneacetone)palladium(0), a phosphine ligand such as 4,5-bis(diphenylphosphino)-9,9-dimethylxanthene and a base such as cesium carbonate in a solvent such as 1,4-dioxan with heating.

A compound of formula (VII) wherein $R^1$, $R^2$ and $R^5$ are as defined in formula (I), may be prepared by treatment of 2-chloro-5-bromopyrimidine with one equivalent of an appropriate aniline of formula (VIII) in a suitable solvent such as an alcohol and heating in a sealed tube under microwave irradiation.

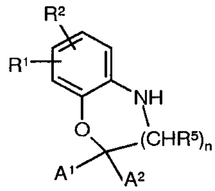

(VIII)

A compound of formula (I), wherein $R^1$, $R^2$, $R^3$, $R^4$, $R^5$, $A^1$ and $A^2$ are as defined for formula (I), X is a carbon atom and Q is a nitrogen atom, may be prepared from a compound of formula (VII) wherein X is a carbon atom by treatment with an appropriate aniline in the presence of a suitable catalyst such as *tris*(dibenzylideneacetone)-palladium(0), a phosphine ligand such as 4,5-*bis*(diphenylphosphino)-9,9-dimethylxanthene and a base such as cesium carbonate in a solvent such as 1,4-dioxan with heating.

A compound of formula (VII) wherein X is a carbon atom, may be prepared by treatment of 2-chloro-5-bromopyridine with one equivalent of an appropriate aniline in a suitable solvent such as an alcohol and heating in a sealed tube under microwave irradiation.

Compounds of formula (VIII) may be prepared by methods well known to those skilled in the art.

During the synthesis of the compounds of formula (I) labile functional groups in the intermediate compounds, e.g. hydroxyl and amino groups, may be protected. The protecting groups are removed during the synthesis of the compounds of formula (I). A comprehensive discussion of the ways in which various labile functional groups may be protected and methods for cleaving the resulting protected derivates is given in for example *Protective Groups in Organic Chemistry,* T.W. Greene and P.G.M. Wuts (Wiley-Interscience, New York, 2nd edition, 1991).

It will be appreciated by someone skilled in the art that by using the methods described above in various combinations it will be possible to synthesise other derivatives encompassed in the general formula (I).

It will also be appreciated that the cyclic aniline and boronic acid building blocks used in the synthesis of compounds of general formula (I) are either commercially available or can be synthesised by methods known in the art.

The pharmaceutically effective compounds of formula (I) may be administered in conventional dosage forms prepared by combining a compound of formula (I) ("active ingredient") with standard pharmaceutical carriers or excipients according to conventional procedures well known in the art. The procedures may involve mixing, granulating and compressing or dissolving the ingredients as appropriate to the desired preparation.

**Thus, in a second aspect, the present invention provides a pharmaceutical composition** comprising a compound of formula (I), or a pharmaceutically acceptable salt or prodrug thereof, together with one or more pharmaceutically acceptable carriers or excipients.

The active ingredient or pharmaceutical composition can be administered simultaneously, separately or sequentially with another appropriate treatment for the amyloid-related disease being treated.

The active ingredient or pharmaceutical composition may be administered to a subject by any of the routes conventionally used for drug administration, for example they may be adapted for oral (including buccal, sublingual), nasal (including inhalation) or parenteral (including subcutaneous, intramuscular, intravenous or intradermal) administration to mammals including humans. The most suitable route for administration in any given case will depend upon the particular compound or pharmaceutical composition, the subject, and the nature and composition and severity of the disease and the physical condition of the subject. Such compositions may be prepared by any method known in the art of pharmacy, for example by bringing into association the active ingredient with the carrier(s) or excipient(s). Preferred routes of administration are oral ad intravenous.

Pharmaceutical compositions adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

Tablets and capsules for oral administration may be in unit dose presentation form , and may contain conventional excipients such as binding agents, for example syrup, acacia, gelatin, sorbitol, tragacanth, or polyvinylpyrrolidine ; filler, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting

agents such as sodium lauryl sulphate. The tablets may be coated according to methods well known in normal pharmaceutical practice. Oral liquid preparations may be in the form of, for example, aqueous or oily suspensions, solutions, emulsions syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, acacia; non-aqueous vehicles (which may include edible oils), for example almond oil, oily esters such as glycerine, propylene glycol, or ethyl alcohol; preservatives, for example methyl or propyl *p*-hydoxybenzoate or sorbic acid, and, if desired, conventional flavouring or colouring agents.

Pharmaceutical compositions adapted for controlled or sustained release may be administered by injection, for example by the subcutaneous route.

Pharmaceutical compositions adapted for nasal administration wherein the carrier is a solid include coarse powder having a particle size for example in the range of 20-500 microns which is administered by rapid inhalation through the nasal passage from a container of the powder held close to the nose. Suitable compositions wherein the carrier is a liquid, for administration as a nasal spray or as nasal drops, include aqueous or oil solutions of an active ingredient.

Pharmaceutical compositions adapted for administration by inhalation include fine particle dusts or mists which may be generated by means of various types of metered dose pressurise aerosols, nebulizers or insufflators.

Pharmaceutical compositions adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The compositions may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solution and suspensions may be prepared from sterile powders, granules and tablets.

For parenteral administration, fluid unit dosage forms are prepared utilising the active ingredient and a sterile vehicle, water being preferred. The active ingredient, depending on the vehicle and concentration used, can be either suspended or dissolved in the vehicle. In preparing solutions the active ingredient can be dissolved in water for injection and filter sterilised before filling into a suitable vial or ampoule and sealing.

Advantageously, agents such as local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the active ingredient is suspended in the vehicle instead of being dissolved and sterilisation cannot be accomplished by filtration. The active ingredient can be sterilised by exposure to ethylene oxide before suspending in the sterile vehicle. Advantageously, a surfactant or wetting agent is included in the composition to facilitate uniform distribution of the active ingredient.

The pharmaceutical compositions according to the invention are preferably adapted for oral, intravenous or subcutaneous administration.

It should be understood that in addition to the ingredients particularly mentioned above, the compositions may also include other agents conventional in the art having regard to the type of formulation in question, for example those suitable for oral administration may include flavouring agents. They may also contain therapeutically active agents in addition to the compounds of the present invention. Such carriers may be present as from about 1% up to about 98% of the formulation. More usually they will form up to about 80% of the formulation.

The compositions may contain from 0.1% by weight, preferably from 10-60% by weight, of the active material, depending on the method of administration.

Pharmaceutical compositions may be presented in unit dose forms containing a predetermined amount of active ingredient per dose. Such a unit may contain for example 0.1mg/kg to 100mg/kg, more preferably 0.1mg/kg to 10mg/kg depending on the condition being treated, the route of administration and the age, weight and condition of the patient. Preferred unit dosage compositions are those containing a daily dose or sub-dose, as herein above recited, or an appropriate fraction thereof, of an active ingredient.

It will be recognised by one of skill in the art that the optimal quantity and spacing of individual dosages of compounds in the first and second aspects of the invention will be determined by the nature and extent of the condition being treated the form, route and site of administration, and the particular subject being treated, and that such optimums can be determined by conventional techniques. It will also be appreciated by one of skill in the art that the optimal course of treatment, i.e., the number of doses of the aforementioned compounds given per day for a defined number of days, can be ascertained by those skilled in the art using conventional course of treatment determination tests.

Depending on the route of administration, the chemical compound or composition may be required to be coated

in a material to protect it from the action of enzymes, acids and other natural conditions which may inactivate it.

In order to administer the chemical compound or composition by other than parenteral administration, it may be coated by, or administered with, a material to prevent its inactivation. For example, it may be administered in an adjuvant, co-administered with enzyme inhibitors or in liposomes. Adjuvant is used in its broadest sense and includes any immune stimulating compound such as interferon. Adjuvants contemplated herein include resorcinols, non-ionic surfactants such as polyoxyethylene oleyl ether and n-hexadecyl polyethylene ether.

Liposomes include water-in-oil-in-water CGF emulsions as well as conventional liposomes.

The active chemical compound or composition may also be administered parenterally or intraperitoneally. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical compositions or formulations suitable for injectable use include sterile aqueous solutions (where water soluble) or dispersions and sterile powders for the extemporaneous preparation of sterile injectable solutions or dispersion. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene gloycol, and the like), suitable mixtures thereof, and vegetable oils. The proper fluidity can be maintained, for example, by the use of a coating such as lecithin, by the maintenance of the required particle size in the case of dispersion and by the use of superfactants.

The prevention of the action of microorganisms can be brought about by various antibacterial and antifungal agents, for example, parabens, chlorobutanol, phenol, sorbic acid, thirmerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active chemical compound or composition in the required amount in the appropriate solvent with various of the other ingredients enumerated above, as required, followed by filtered sterilisation. Generally, dispersions are prepared by incorporating the sterilised active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the chemical compound or composition is suitably protected as described above, it may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. The amount of active compound in such therapeutically useful compositions is such that a suitable dosage will be obtained.

The tablets, troches, pills, capsules and the like may also contain the following: a binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavouring agent such as peppermint, oil of wintergreen, or cherry flavouring. When the dosage unit form is a capsule, it may contain, in addition to materials of the above type, a liquid carrier.

Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound may be incorporated into sustained-release preparations and formulations.

As used herein "pharmaceutically acceptable carrier and/or diluent" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of

compounding such as active material for the treatment of disease in living subjects having a diseased condition in which bodily health is impaired.

The principal active ingredients are compounded for convenient and effective administration in effective amounts with a suitable pharmaceutically acceptable carrier in dosage unit form. In the case of compositions containing supplementary active ingredients, the dosages are determined by reference to the usual dose and manner of administration of the said ingredients.

In other aspects, the present invention provides:

1. A compound of the invention for use in the treatment of an amyloid-related disease. In particular, the medicament is for the treatment of:

   a) any form of Alzheimer’s disease (AD or FAD);

   b) any form of mild cognitive impairment (MCI) or senile dementia;

   c) Down’s syndrome;

   d) cerebral amyloid angiopathy, inclusion body myositis, hereditary cerebral hemorrhage with amyloidosis (HCHWA, Dutch type), or age-related macular degeneration (ARMD);

   e) fronto-temporal dementia;

   f) any form of Parkinson’s disease (PD) or dementia with Lewy bodies;

   g) Huntington’s disease (HD), dentatorubral pallidoluysian atrophy (DRPLA), spinocerebellar ataxia (SCA, types 1, 2, 3, 6 and 7), spinal and bulbar muscular atrophy (SBMA, Kennedy’s disease), or any other polyglutamine disease;

   h) Creutzfeldt-Jakob disease (CJD), bovine spongiform encephalopathy (BSE) in cows, scrapie in sheep, kuru, Gerstmann-Straussler-Scheinker disease (GSS), fatal familial insomnia, or any other transmissible encephalopathy that is associated with the aggregation of prion proteins;

   i) amyotrophic lateral sclerosis (ALS) or any other form of motor neuron disease;

   j) familial British dementia (FBD) or familial Danish dementia (FDD);

   k) hereditary cerebral hemorrhage with amyloidosis (HCHWA, Icelandic type);

   l) type II diabetes (adult onset diabetes, or non-insulin dependent diabetes mellitus, NIDDM);

   m) dialysis-related amyloidosis (DRA) or prostatic amyloid;

   n) primary systemic amyloidosis, systemic AL amyloidosis, or nodular AL amyloidosis;

   o) myeloma associated amyloidosis;

   p) systemic (reactive) AA amyloidosis, secondary systemic amyloidosis, chronic inflammatory disease, or familial Mediterranean fever;

   q) senile systemic amyloidosis, familial amyloid polyneuropathy, or familial cardiac amyloid;

   r) familial visceral amyloidosis, hereditary non-neuropathic systemic amyloidosis, or any other lysozyme-related amyloidosis;

   s) Finnish hereditary systemic amyloidosis;

   t) fibrinogen $\alpha$-chain amyloidosis;

u) insulin-related amyloidosis;

v) medullary carcinoma of the thyroid;

w) isolated atrial amyloidosis;

x) any form of cataract; and

y) any other amyloid-related disease that is associated with the misfolding or aggregation of a specific target amyloid-forming protein or peptide into toxic soluble oligomers, protofibrils, ion channels, insoluble amyloid fibres, plaques or inclusions.

**Examples**

**Intermediate 1: 2,4-Dihydroxy-5-methoxypyrimidine**

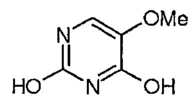

A mixture of methyl methoxyacetate (104g. 1.0mol) and ethyl formate (74g. 1.0mol) was added dropwise to a stirred suspension of sodium (23g. 1.0mol) in toluene (300mL), the temperature being kept below 30°C. After 24h the toluene layer was decanted and to the crude, viscous methyl sodio-β-hydroxy-α-methoxyacrylate were added ethanol (150mL) and urea (60g. 1.0mol). The mixture was stirred for 1h. at room temperature, and then heated under reflux for 5h. After cooling, the solid was collected and dissolved in water (500mL) and the solution was neutralized with 6N aq. hydrochloric acid. The resultant precipitate was collected by filtration and dried at 100°C. This material, 2,4-dihydroxy-5-methoxypyrimidine, (47g, 33%) was used directly in the next stage.
**NMR:** $\delta_H$($d_6$-DMSO) 3.57 (3H, s), 7.00 (1H, s), 10.45 (1H, br) and 11.20 (1H, br).

**Intermediate 2: 2,4-Dichloro-5-methoxypyrimidine**

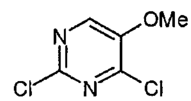

2,4-Dihydroxy-5-methoxypyrimidine (45g, 0.316mol), phosphorous oxychloride (225mL, 0.88mol), and N,N-dimethylaniline (45mL, 0.391mol) were heated under reflux for 2h. The mixture was poured onto crushed ice (80 g.) and the product extracted into diethyl ether. Recrystallisation from light petroleum (b.p: 40-60°C) gave 2,4-dichloro-5-methoxypyrimidine (43g, 75%).
Mass: (ES+) 179 $(M+H)^+$

**Intermediate 3: 2-Chloro-5-methoxypyrimidine**

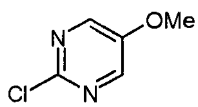

2,4-Dichloro-5-methoxypyrimidine (43g, 0.24mol), zinc dust (86g, 1.32mol), ethanol (200 mL) and water (200 mL) were heated under reflux for 4h. The hot mixture was filtered and the ethanol was removed under reduced pressure.

After cooling, the product was extracted into diethyl ether. Recrystallisation from light petroleum (b.p.: 40-60°C) gave 2-chloro-5-methoxypyrimidine (20g, 58%).
**Mass:** (ES+) 145 $(M+H)^+$
**NMR:** $\delta_H$ ($d_6$-DMSO) 3.92 (3H, s) and 8.55 (2H, s).

**Intermediate 4: 2-Chloro-5-hydroxypyrimidine**

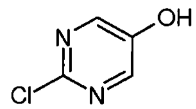

To a solution of 2-chloro-5-methoxypyrimidine (10g, 0.069mol) in dichloromethane (84mL) was added dropwise boron tribromide (104mL, 1M solution in dichloromethane) at -78°C. The mixture was stirred at room temperature for 20h and then methanol (150mL) was added dropwise at -78°C. The reaction mixture was concentrated under reduced pressure and the pH adjusted to 5 with aq. sodium hydroxide solution. The mixture was extracted with ethyl acetate and washed with water and brine. The organic phase was dried ($MgSO_4$) and the solvent removed under reduced pressure to give 2-chloro-5-hydroxypyrimidine (5.7g, 61%).
**Mass:** (ES-) 129
**NMR:** $\delta_H$ ($d_6$-DMSO) 8.27 (2H, s) and 10.85 (1H, br s).

**Intermediate 5: 3-Morpholin-4-ylphenyl boronic acid**

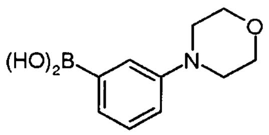

1,3-Dibromobenzene (50g, 0.21mol), morpholine (15.89mL, 0.19mol) and anhydrous toluene (200mL) were added to a flask by syringe under argon. The solution was thoroughly mixed before R-BINAP (1.32g, 0.0021mol) and *tris*(dibenzylideneacetone) palladium (0) (0.640g, 0.006mol) were added and finally DBU (25.8mL, 0.17mol) was added via syringe. The reaction mixture was stirred at 60°C. Sodium *tert*-butoxide (30.55 g, 0.32mol) was added and the reaction was heated to 100°C overnight. The suspension was diluted with ethyl acetate, filtered through Celite and washed with water and brine. The organic phase was dried ($MgSO_4$) and the solvent removed under reduced pressure. The crude product was purified by column chromatography on silica eluting with ethyl acetate:hexane, 1:1, to afford 4-(3-bromophenyl)morpholine as yellow oil (36.5g, 71%).
**Mass:** (ES+) 242 $(M+H)^+$
To a stirred mixture of 4-(3-bromophenyl)morpholine (16.5g, 0.068mol) in dry THF (300mL) was added n-butyl lithium (98.1mL, 0.102mol, 1.6M solution in n-hexane) dropwise at -70°C to -78°C. The mixture was stirred at same temperature for 2h. To this reaction mixture was added tri-isopropyl borate (30.5mL, 0.150mol) dropwise whilst maintaining the temperature at -70°C to -78°C. The dry ice-acetone bath was removed and the mixture was allowed to warm to room temperature and stirred at room temperature overnight. The mixture was poured into a saturated solution of ammonium chloride and water added and the reaction mixture was extracted with ethyl acetate. The organic phase was dried ($MgSO_4$) and the solvent removed under reduced pressure. The residue was triturated with n-hexane and diethyl ether to afford 3-morpholin-4-yl-phenylboronic acid (12g, 85%) as an off white solid.
**Mass:** (ES+) 208 $(M+H)^+$
**NMR:** $\delta_h$ ($d_6$-DMSO) 3.05 (4H, m), 3.72 (4H, m), 6.95 (1H, d), 7.18 (1H, t), 7.21 (1H, d), 7.38 (1H, br s) and 7.94 (2H, s).

**Intermediate 6: (2-Fluoro-6-nitrophenoxy)acetic acid, methyl ester**

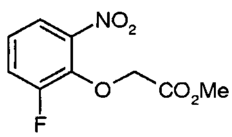

A stirred mixture of 2-fluoro-6-nitrophenol (2.2g, 0.0140mol), methyl bromoacetate (3.2mL, 0.0177 mol), $K_2CO_3$ (4 g, 0.0184mol) in acetone (100mL) was heated at 65°C for 3h. The reaction mixture was concentrated under vacuum and the resultant solid was dissolved in dichloromethane. Water was then added and the mixture was extracted with dichloromethane. The organic phase was dried ($MgSO_4$) and the solvent removed under reduced pressure. The residue was triturated with diethyl ether to afford (2-fluoro-6-nitrophenoxy)acetic acid, methyl ester as brown oil (1.44g, 98%) which was used directly in the next stage.
**Mass:** (ES+) 198 $(M+H)^+$

**Intermediate 7:** 8-Fluoro-4*H*-benzo[1,4]oxazin-3-one

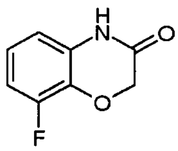

A stirred mixture of (2-fluoro-6-nitrophenoxy)acetic acid, methyl ester (1.44g, 0.0073mol) and iron powder (2.44g, 0.0438mol) in acetic acid (50mL) was stirred at room temperature overnight. The pH of the reaction mixture was adjusted to 7 by using sodium bicarbonate and sodium hydroxide solutions. The mixture was diluted with ethyl acetate. Water was added and the mixture was extracted with dichloromethane. The organic phase was dried ($MgSO_4$) and the solvent removed under reduced pressure. The residue was triturated with diethyl ether to afford 8-fluoro-4*H*-benzo[1,4]oxazin-3-one as a yellow solid (1.0g, 82%).
**NMR:** $\delta_H$ ($d_6$-DMSO) 4.63 (2H, s), 6.70 (1H, d), 6.88 (2H, m) and 10.88 (1H, br s).

**Intermediate 8: 8-Fluoro-3,4-dihydro-2H-benzo[1,4]oxazine**

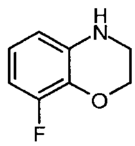

To a stirred mixture of 8-fluoro-2*H*-1, 4-benzoxazin-3(4*H*)-one (1.0g, 0.0059mol) in THF (25mL) was added lithium aluminium hydride (7mL, 1M solution in THF) dropwise at 0°C to -5°C. The reaction mixture was stirred at room temperature for 2h and then poured onto ice and extracted with ethyl acetate. The organic phase was dried ($MgSO_4$) and the solvent removed under reduced pressure. The residue was triturated with diethyl ether to afford 8-fluoro-3,4-dihydro-2H-benzo[1,4]oxazine (0.8g, 87%) as a dark brown solid.
**Mass:** (ES+) 154 $(M+H)^+$
**NMR:** $\delta_H$ ($d_6$-DMSO) 3.27 (2H, m), 4.11 (2H, m), 6.05 (1H, br s), 6.34 (2H, m) and 6.58 (1H, m).

**Intermediate 9: 4-[3-(2-Chloropyrimidin-5-yloxy)phenyl]morpholine**

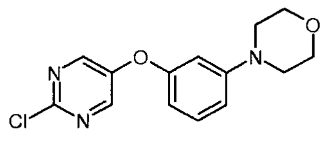

A mixture of 2-chloro-5-hydroxypyrimidine (15g, 0.115mol), 3-morpholinylphenyl boronic acid (24g, 0.115mol), copper (II) acetate (21g, 0.115mol), triethylamine (79.5mL, 0.402mol) and powdered 4Å molecular sieves in dichloromethane (600mL) was stirred under air for 3 days. A calcium chloride guard tube was used to protect the reaction from moisture. The suspension was diluted with dichloromethane, filtered and washed with water and brine. The organic phase was dried ($MgSO_4$) and the solvent removed under reduced pressure. The crude product was purified by column chromatography on silica (ethyl acetate:hexane 1:1) to afford 4-[3-(2-chloropyrimidin-5-yloxy)phenyl]morpholine (8.0g, 24%) as colourless oil.
**Mass:** (ES+) 292 $(M+H)^+$
**NMR:** $\delta_H$ ($d_6$-DMSO) 3.15 (4H, m), 3.84 (4H, m), 6.50 (1H, d), 6.58 (1H, s), 6.75 (1H, d), 7.28 (1H, t) and 8.35 (2H, s).

**Intermediate 10: 4-[3-(6-Chloropyridin-3-yloxy)phenyl]morpholine**

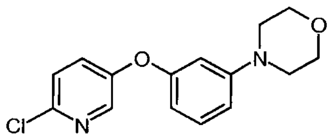

A mixture of 2-chloro-5-hydroxypyridine (2g, 0.0154mol), 3-morpholin-4-ylphenylboronic acid (3.2g, 0.0154mol), copper (II) acetate (2.8g, 0.0154mol), triethylamine (10.8mL, 0.077mol) and powdered 4Å molecular sieves in dichloromethane (90mL) was stirred under air for 3 days. A calcium chloride guard tube was used to protect the reaction from moisture. The reaction mixture was diluted with dichloromethane, filtered and washed with water and brine. The organic phase was dried ($MgSO_4$) and the solvent removed under reduced pressure. The crude product was purified by column chromatography on silica gel (ethyl acetate:hexane 1:1) to afford 4-[3-(6-chloropyridin-3-yloxy)phenyl] morpholine (1.0g, 22%) as colourless oil.
Mass: (ES+) 291 $(M+H)^+$

**Intermediate 11: (4-fluoro-6-nitrophenoxy)acetic acid, methyl ester**

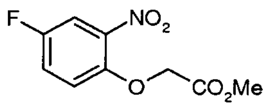

A stirred mixture of 4-fluoro-6-nitrophenol (2.2g, 0.0140mol), methyl bromoacetate (3.2g, 0.0177mol), $K_2CO_3$ (4g, 0.0184mol) in acetone (100mL) was heated at reflux for 3h. The mixture was concentrated under vacuum and the resultant solid was dissolved in dichloromethane. Water was then added and the reaction mixture was extracted with dichloromethane. The organic phase was dried ($MgSO_4$) and the solvent removed under reduced pressure. The residue was triturated with diethyl ether to afford (4-fluoro-6-nitrophenoxy)acetic acid, methyl ester (1.3g, 88%) as brown oil which was used directly in the next stage.
**Mass:** (ES+) 198 $(M+H)^+$

**Intermediate 12: 6-Fluoro-4*H*-benzo[1,4]oxazin-3-one**

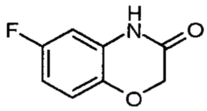

A mixture of (4-fluoro-6-nitrophenoxy)acetic acid, methyl ester (1.3g, 0.0065mol), iron powder (2.2g, 0.0395mol) in acetic acid (45mL) was stirred at room temperature overnight. The pH of the reaction mixture was adjusted to 7 by using sodium bicarbonate and sodium hydroxide solutions. The mixture was diluted with ethyl acetate. Water was added and the mixture was extracted with dichloromethane. The organic phase was dried ($MgSO_4$) and the solvent removed under reduced pressure. The residue was triturated with diethyl ether to afford 6-fluoro-4*H*-benzo[1,4]oxazin-3-one (0.9g, 82%) as a yellow solid which was used directly in the next stage.
**Mass:** (ES-) 166

**Intermediate 13: 6-Fluoro-3,4-dihydro-2H-benzo[1,4]oxazine**

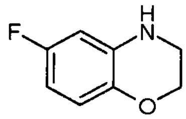

To a stirred mixture of 6-fluoro-2*H*-1,4-benzoxazin-3(4*H*)-one (0.9g, 0.0053mol) in THF (23mL) was added lithium aluminium hydride (6.5mL,1M solution in THF) dropwise at 0°C to -5°C. The reaction mixture was stirred at room temperature for 2h. The mixture was poured onto ice and extracted with ethyl acetate. The organic phase was dried ($MgSO_4$) and the solvent removed under reduced pressure. The residue was triturated with diethyl ether to afford 6-fluoro-3,4-dihydro-2H-benzo[1,4]oxazine (0.5g, 61 %) as a dark brown solid.
**NMR:** $\delta_H$ ($d_6$-DMSO) 3.25 (2H, m), 4.05 (2H, m), 6.03 (1H, br s), 6.17 (1H, dt), 6.31 (1H, dd) and 6.57 (1H, dd).

**Intermediate 14: 1-(3-Bromophenyl)-4-methylpiperazine**

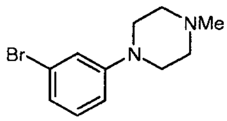

1,3-Dibromobenzene (10g, 0.0423mol), N-methylpiperazine (3.2mL, 0.0296mol) and anhydrous toluene (40mL) were added to a flask by syringe, under argon. The resultant solution was thoroughly mixed before R-BINAP (0.264g, 0.00042mol) and *tris*(dibenzylideneacetone)palladium (0) (0.128g, 0.00013mol) were delivered and then DBU (5.16mL, 0.0345mol) was added *via* syringe. The reaction mixture was stirred at 60°C for 5 minutes. Sodium *tert*-butoxide (6.11 g, 0.0635mol) was added and the reaction heated to 100° C overnight. The suspension was diluted with ethyl acetate, filtered through Celite and washed with water and brine. The organic phase was dried ($MgSO_4$) and the solvent removed under reduced pressure. The crude product was purified by column chromatography on silica eluting with dichloromethane: methanol (19:1) to afford 1-(3-bromophenyl)-4-methylpiperazine (5.6g, 52%) as yellow oil.
**Mass:** (ES+) 256 $(M+H)^+$

**Intermediate 15: 3-(4-Methylpiperazin-1-yl)phenyl boronic acid**

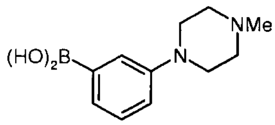

To a stirred mixture of 1-(3-bromophenyl)-4-methylpiperazine (5.6g, 0.0222mol) in dry THF (80mL) was added

n-butyl lithium (33.5mL, 0.0333mol, 1.6M solution in n-hexane) dropwise at -70°C to -78°C. The reaction mixture was stirred at same temperature for 2h. To this mixture was added tri-isopropyl borate (11.28mL, 0.0489mol) dropwise whilst maintaining the temperature at -70°C to -78°C. The dry ice-acetone bath was removed and the mixture was allowed to warm to room temperature and stirred at this temperature overnight. The mixture was poured into the saturated solution of ammonium chloride and added water and the reaction mixture was extracted with ethyl acetate. The organic phase was dried ($MgSO_4$) and the solvent removed under reduced pressure. The residue was triturated with n-hexane and diethyl ether to afford 3-(4-methylpiperazinyl)phenyl boronic acid (1.26g, 26%) as an oil.
**Mass:** (ES+) 221 $(M+H)^+$

**Intermediate 16: 1-[3-(2-Chloropyrimidin-5-yloxy)phenyl]-4-methylpiperazine**

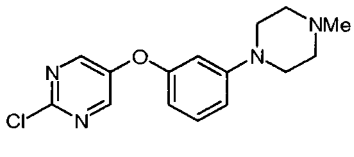

A mixture of 2-chloro-5-hydroxypyrimidine (0.711g, 0.00545mol), 3-(4-methylpiperazin-1-yl)phenyl boronic acid (1.5g, 0.00545mol), copper (II) acetate (0.99g, 0.00545mol), triethylamine (3.8mL, 0.0272mol) and powdered 4Å molecular sieves in dichloromethane (40mL) was stirred under air for 3 days. The suspension was diluted with dichloromethane, filtered and washed with water and brine. The organic phase was dried ($MgSO_4$) and the solvent removed under reduced pressure. The crude product was purified by column chromatography on silica eluting with dichloromethane:methanol (19:1) to afford 1-[3-(2-chloropyrimidin-5-yloxy)phenyl]-4-methylpiperazine (0.68g, 41%) as colourless oil.
**Mass:** (ES+) 305 $(M+H)^+$

**Intermediate 17: 9-Fluoro-2,3,4,5-tetrahydrobenzo[b][1,4]oxazepine**

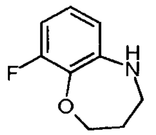

To a stirred mixture of 2-fluoro-6-aminophenol (2g, 0.0157mol), $K_2CO_3$ (10.8 g, 0.0784 mol) in DMF (30mL) was added 1,3-dibromopropane (4.75g, 0.0235mol) dropwise at room temperature. The mixture was stirred at 125°C for 24h and then poured onto ice and extracted with ethyl acetate. The organic phase was dried ($MgSO_4$) and the solvent removed under reduced pressure. The crude product was purified by column chromatography using ethyl acetate:hexane (7:3) as the mobile phase to afford 9-fluoro-2,3,4,5-tetrahydrobenzo[b][1,4]oxazepine (0.3g, 11%) as brown oil.
**Mass:** (ES+) 168 $(M+H)^+$
**NMR:** N-388/ST-VIII/94-amine/47

**Intermediate 18: 2,3-Dihydro-1H-pyrido[2,3-b][1,4]oxazine**

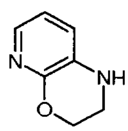

To a stirred mixture of 3-aminopyridin-2-ol (1.0g, 9.08mmol), $K_2CO_3$ (6.27g, 45.46mmol) in DMF (10mL) was added 1,2-dibromoethane (2.53 g, 13.6mmol) dropwise at room temperature. The mixture was stirred at 80°C for 24h and then poured onto ice, extracted with ethyl acetate and washed with water and brine. The organic phase was dried

($MgSO_4$) and the solvent removed under reduced pressure. The crude product was purified by column chromatography using ethyl acetate:hexane (7:3) as the mobile phase to afford 2,3-dihydro-1H-pyrido[2,3-b][1,4]oxazine (100mg, 8%) as brown oil.
Mass: (ES+) 137 $(M+H)^+$

**Intermediate 19: (R)-4-(3-Bromophenyl)-3-methylmorpholine**

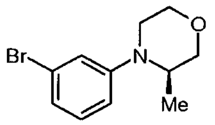

1, 3-Dibromobenzene (1.97mL, 0.0162mol), (R)-3-methylmorpholine (1.5 g, 0.0146 mol) and anhydrous toluene (50mL) were added to a flask by syringe under argon. The solution was thoroughly mixed before R-BINAP (0.101 g, 0.00016mol), *tris* (dibenzyl-ideneacetone) palladium (0) (0.049 g, 0.000053 mol) were added and then finally DBU (1.98mL, 0.0132 mol) was added via syringe. The reaction mixture was stirred at 6°C. Sodium t-butoxide (2.34 g, 0.0244mol) was added and the reaction mixture was heated at 100°C overnight. The suspension was diluted with dichloromethane, filtered through celite and washed with water and brine. The organic phase was dried ($MgSO_4$) and the solvent removed under reduced pressure. The crude product was purified by flash chromatography, using 0-30% ethyl acetate:hexane as eluent, to give (R)-4-(3-bromo-phenyl)-3-methylmorpholine (0.75g) as yellow oil.
**Mass:** (ES+) 256 $(M+H)^+$
**NMR:** $\delta_H$ ($d_6$-DMSO) 1.00 (3H, d), 2.98 (1H, dt), 3.21(1H, br d), 3.52 (1H, dt), 3.67 (2H, m), 3.90 (2H, br s), 6.89 (2H, br d), 7.01 (1h m) and 7.15 (1H, t).

**Intermediate 20: (R)-3-(3-Methyl-morpholin-4-yl)phenylboronic acid**

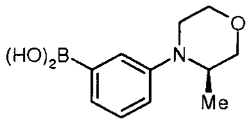

To a stirred mixture of (S)-4-(3-bromophenyl)-3-methylmorpholine (0.745g, 0.00291mol) in dry THF (20mL) was added n-BuLi (6.0 mL, 0.0096mol, 1.6 M solution in n-hexane) dropwise at -70°C to -78°C. The mixture was stirred at the same temperature for 2h. To this mixture was added tri-isopropyl borate (2.0mL, 0.00873mol) dropwise whilst maintaining the temperature. The dry ice acetone bath was removed and the mixture was allowed to warm to room temperature and stirred at room temperature overnight. The mixture was poured into saturated aqueous ammonium chloride, water was added and the reaction mixture was extracted with ethyl acetate. The organic phase was dried ($MgSO_4$) and the solvent removed under reduced pressure and below 40°C to provide (S)-3-(3-methyl-morpholin-4-yl)phenylboronic acid (0.70g) which was of sufficient purity to use directly in the next step.
**Mass:** (ES+) 221 $(M+H)^+$

**Intermediate 21: (R)-4-[3-(2-Chloropyrimidin-5-yloxy)phenyl]-3-methylmorpholine**

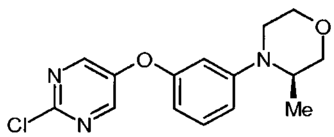

A mixture of 2-chloro-5-hydroxypyrimidine (0.295g, 2.261mmol), (S)-3-(3-methylmorpholin-4-yl)phenylboronic acid (0.5g, 2.261mmol), copper (II) acetate (0.410g, 2.261mmol), triethylamine (1.6mL, 11.3mmol) and powdered 4Å

molecular sieves in dichloromethane (50mL) was stirred under air for 3 days. A calcium chloride guard tube was used to protect the reaction from moisture. The suspension was diluted with dichloromethane, filtered and washed with water and brine. The organic phase was dried ($MgSO_4$) and the solvent removed under reduced pressure. The crude product was purified by flash chromatography, using 0-15% ethyl acetate:hexane as eluent, to give (R)-4-{3-[(2-chloropyimidin-5-yl)oxy]phenyl}3-methylmorpholine (0.258g) as a brown oil.
**Mass:** (ES+) 305 $(M+H)^+$

**Intermediate 22: (S)-4-(3-Bromophenyl)-3-methylmorpholine**

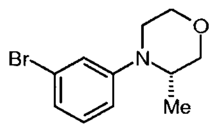

A mixture of 1, 3-dibromobenzene (0.59mL, 4.95mmol), (R)-3-methylmorpholine (0.5g, 4.95mmol), R-BINAP (0.231g, 0.37mmol), tris(dibenzylideneacetone)-palladium (0) (0.113g, 0.123mmol), sodium t-butoxide (0.570 g, 5.94mmol) in anhydrous toluene (10mL) in a 35mL sealed tube was heated at 85-95°C overnight. The suspension was diluted with dichloromethane, filtered through celite and washed with water and brine. The organic phase was dried ($MgSO_4$) and the solvent removed under reduced pressure. The crude product was purified by flash chromatography, using 0-15% ethyl acetate:hexane as eluent, to give (S)-4-(3-bromophenyl)-3-methylmorpholine (0.953g) as a yellow oil.
**Mass:** (ES+) 256 $(M+H)^+$
**NMR:** $\delta_H$ ($d_6$-DMSO) 1.00 (3H, d), 2.98 (1H, dt), 3.21(1H, br d), 3.52 (1H, dt), 3.67 (2H, m), 3.90 (2H, br s), 6.89 (2H, br d), 7.01 (1h m) and 7.15 (1H, t).

**Intermediate 23: (S)-3-(3-Methyl-morpholin-4-yl)phenylboronic acid**

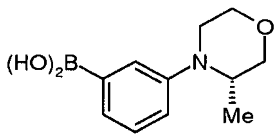

To a stirred mixture of (R)-4-(3-bromophenyl)-3-methylmorpholine (0.953g, 0.00372mol) in dry THF (20mL) was added n-BuLi (7.7mL, 0.00123mol), 1.6 M solution in n-hexane) dropwise at -70°C to -78°C. The mixture was stirred at same temperature for 2h. To this mixture was added tri-isopropyl borate (2.6mL, 0.0113mol) dropwise whilst maintaining the temperature. The dry ice acetone bath was removed and the mixture was allowed to warm to room temperature and stirred at room temperature for overnight. The reaction mixture was poured into saturated aqueous ammonium chloride, water was added and the mixture was extracted with ethyl acetate. The organic phase was dried ($MgSO_4$) and the solvent removed under reduced pressure and below 40°C. The residue was triturated with hexane to give (S)-3-(3-methyl-morpholin-4-yl)phenylboronic acid (0.70g, 51%) which was used directly in the next stage.
**Mass:** (ES+) 221 $(M+H)^+$

**Intermediate 24: (S)-4-[3-(2-Chloropyrimidin-5-yloxy)phenyl]-3-methylmorpholine**

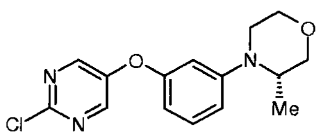

A mixture of 2-chloro-5-hydroxypyrimidine (0.250g, 0.00191mol), R-3-(3-methylmorpholin-4-yl)phenyl boronic acid (0.423 g, 0.00191mol), copper (II) acetate (0.347g, 0.00191mol), triethylamine (1.33mL, 0.0096mol) and powdered

4Å molecular sieves in dichloromethane (30mL) was stirred under air for 3 days. A calcium chloride guard tube was used to protect the reaction from moisture. The suspension was diluted with dichloromethane, filtered and washed with water and brine. The organic phase was dried ($MgSO_4$) and the solvent removed under reduced pressure. The crude product was purified by flash chromatography, using 0-15% ethyl acetate:hexane as eluent, to afford (S)-4-[3-(2-chloropyrimidin-5-yloxy)phenyl]-3-methylmorpholine (0.175g, 30%) as brown oil.
Mass: (ES+) 305 $(M+H)^+$

**Intermediate 25: 2-Chloro-5-(3-methoxyphenoxy)pyrimidine**

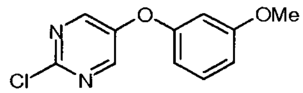

A mixture of 2-chloro-5-hydroxypyrimidine (1.0g, 7.66mmol), 3-methoxyphenyl boronic acid (1.16g, 7.66mmol), copper (II) acetate (1.39g, 7.66mmol), triethylamine (5.34mL, 7.66mmol) and powdered 4Å molecular sieves in dichloromethane (30mL) was stirred under air for 3 days. A calcium chloride guard tube was used to protect the reaction from moisture. The reaction mixture was diluted with dichloromethane, filtered and washed with water and brine. The organic phase was dried ($MgSO_4$), the solvent removed under reduced pressure and the crude product was purified by flash chromatography, using 0-15% ethyl acetate:hexane as eluent, to provide 2-chloro-5-(3-methoxyphenoxy)-pyrimidine (0.448g, 25%) as yellow oil.
**Mass:** (ES+) 237 $(M+H)^+$
**NMR:** $\delta_H$ ($CDCl_3$) 3.82 (3H, s), 6.63 (2H, m), 6.81 (1H, br d), 7.33 (1H, t) and 8.39 (2H, s).

**Intermediate 26: 2-Chloro-5-benzyloxypyrimidine**

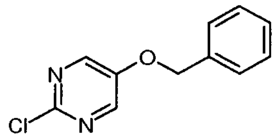

To a stirred mixture of 2-chloro 5-hydroxy pyrimidine (2.0g, 0.01532mol), $K_2CO_3$ (4.23g, 0.0307mol) in dry THF (30mL) was added benzyl chloride (2.6mL, 0.023mol) dropwise at room temperature. The mixture was heated at reflux overnight. The reaction mixture was diluted with dichloromethane and washed with water and brine. The organic phase was dried ($MgSO_4$) and the solvent removed under reduced pressure to provide 2-chloro-5-benzyloxypyrimidine (1.3g) which was of sufficient purity to use directly in the next step.
**Mass:** (ES+) 221 $(M+H)^+$

**Intermediate 27: 4-[5-(Benzyloxy)pyrimidin-2yl]-8-fluoro-3,4-dihydro-2H-benzo-[1,4]oxazine**

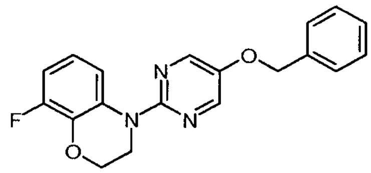

A suspension of 2-chloro-5-benzyloxypyrimidine (1.25g, 0.00567mol), 8-fluoro-3,4-dihydro-2H-benzo[b][1,4]oxazine (868mg, 0.00567mol), *tris*(dibenzylideneacetone)-palladium(0) (360mg, 0.00028mol), 4,5-*bis*(diphenylphosphino)-9,9-dimethylxanthene (328mg, 0.00056mol) and cesium carbonate (3.7g, 0.01134mol) in de-gassed 1,4-dioxane (15mL) was heated at 80°C for 2 days. The reaction mixture was diluted with dichloromethane and washed with water and brine. The organic phase was dried ($MgSO_4$) and the solvent removed under reduced pressure and the crude

product was purified by flash chromatography, using 0-5% ethyl acetate:hexane as eluent, to give 4-(5-(benzyloxy)pyrimidine-2yl)-8-fluoro3,4-dihydro-2H-benzo[1,4]oxazine (1.14g, 60%) as yellow solid.
**Mass:** (ES+) 338(M+H)$^+$

**Intermediate 28: 8-Fluoro-4-[5-(hydroxy)pyrimidin-2yl]-3,4-dihydro-2H-benzo-[1,4]oxazine**

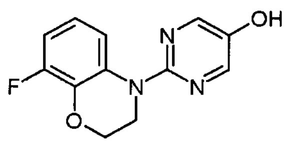

A stirred mixture of 4-[5-(benzyloxy)pyrimidin-2yl]-8-fluoro-3,4-dihydro-2H-benzo-[1,4]oxazine (1.1g), palladium on carbon (0.4g) in dry ethanol was purged with hydrogen at room temperature for 20 minutes. The reaction mixture was filtered through celite and washed with ethanol and the solvent removed under reduced pressure to provide 8-fluoro-4-[5-(hydroxy)pyrimidin-2yl]-3,4-dihydro-2H-benzo[1,4]oxazine (0.818g).
**Mass:** (ES+) 248 (M+H)$^+$

**Intermediate 29: 4-Benzyl-8-fluoro-4H-benzo[1,4]oxazin-3-one**

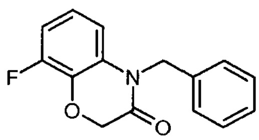

To a stirred mixture of 8-fluoro-4H-benzo[1,4]oxazin-3-one (1g, 0.0059mol) and caesium carbonate (4.8g, 0.0147mol) in 1,4 dioxane (15mL) was added benzyl chloride (0.821g, 0.00649mol) dropwise at 0°C to 5°C. The mixture was heated at 100-110°C for 2h. The reaction mixture was poured onto ice and extracted with ethyl acetate. The organic phase was dried ($MgSO_4$) and the solvent removed under reduced pressure at below 40°C. The residue was triturated with hexane to afford 8-fluoro-4H-benzo-[1,4]oxazin-3-one (1.3g, 85%) as a light brown solid.
**Mass:** (ES+) 258(M+H)$^+$

**Intermediate 30: (±)-4-Benzyl-8-fluoro-3-methyl-3,4-dihydro-2H-benzo[1,4]-oxazine**

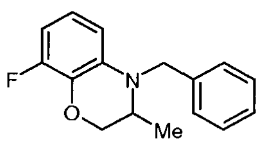

To a stirred mixture of 4-benzyl-8-fluoro-2H-benzo[1,4]oxazin-3-one (1g, 0.0038mol) in THF (15mL) was added $CH_3MgBr$ (5.07mL, 3M solution in THF) dropwise at 0°C to -5°C. The mixture was stirred at room temperature for 2 hr. The reaction mixture was cooled to 0-5°C and acetic acid (10mL) was added followed by $NaBH_4$ (0.361g, 0.0095mol), slowly at 0-5°C. The reaction mixture was stirred at room temperature for 12h and then poured onto ice and extracted with ethyl acetate. The organic phase was dried ($MgSO_4$) and the solvent removed under reduced pressure at below 40°C. The residue was triturated with diethyl ether to afford (±)-4-benzyl-8-fluoro-3-methyl-3,4-dihydro-2H-benzo[1,4]oxazine (0.8g, 80%).
Mass: (ES+) 257 (M+H) $^+$

**Intermediate 31: (±)-8-Fluoro-3-methyl-3,4-dihydro-2H-benzo[1,4]oxazine**

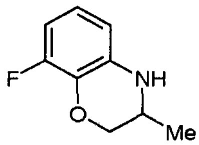

A stirred mixture of (±)-4-benzyl-8-fluoro-3-methyl-3,4-dihydro-2H-benzo[1,4]-oxazine (0.8 g, 0.0031mmol) and palladium on carbon (0.24g, 10%, 50% $H_2O$ w/w) in ethanol was purged with hydrogen at room temperature for 4h. The mixture was filtered through a bed of celite and washed with ethanol and the solvent removed under reduced pressure at below 40°C to give (±)-8-fluoro-3-methyl-3,4-dihydro-2H-benzo[1,4]oxazine as a light yellow solid (0.4g, 77%) which was used directly used for Example 16 without purification.
Mass: (ES+) 167 $(M+H)^+$

**Intermediate 32: 2-Chloro-5-(3-N,N-dimethylaminophenoxy)pyrimidine**

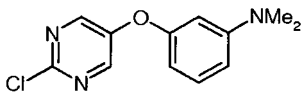

A mixture of 2-chloro-5-hydroxypyrimidine (1.0g, 0.00766mol), 3-N,N-dimethylamino-phenyl boronic acid (1.26g, 0.00766mol), copper (II) acetate (1.39g, 0.00766mol), triethylamine (5.34mL, 0.00766mol) and powdered 4Å molecular sieves in dichloro-methane (30mL) was stirred under air for 3 days. A calcium chloride guard tube was used to protect the reaction from moisture. The suspension was diluted with dichloromethane, filtered and washed with water and brine. The organic phase was dried ($MgSO_4$) and the solvent removed under reduced pressure. The crude product was purified by flash chromatography using 0-15% ethyl acetate: hexane as eluent to obtained 2-chloro-5-(3-N,N-dimethylaminophenyl)pyrimidine (0.448, 25%) as a yellow oil.
**Mass:** (ES+) 250 $(M+H)^+$
**HPLC:** 99.8%
**NMR:** $\delta_H(CDCl_3)$ 2.98 (6H, s), 6.35 (1H, dd), 6.40 (1H, m), 6.60 (1H, dd), 7.25 (1H, t) and 8.38 (2H, s).

**Example 1: 8-Fluoro-4-[5-(3-morpholinophenoxy)pyrimidin-2-yl]-3,4-dihydro-2H-benzo[1,4]oxazine**

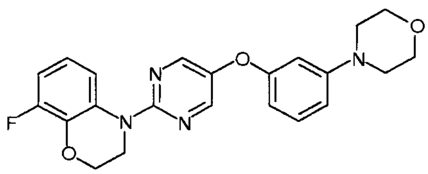

A suspension of 4-[3-(2-chloropyrimidin-5-yloxy)phenyl]morpholine (400mg, 1.37mmol), 8-fluoro-2*H*-1,4-benzoxazin-3(4*H*)-one (210mg, 1.37mmol), *tris*(dibenzylideneacetone)palladium(0) (62mg, 0.067mmol), 4,5-*bis*(diphenylphosphino)-9,9- dimethylxanthene (79mg, 0.137mmol) and cesium carbonate (895mg, 2.75mmol) in degassed 1,4-dioxane (8mL) was heated at 80°C for 2 days. The suspension was diluted with ethyl acetate and washed with water and brine. The organic phase was dried ($MgSO_4$) and the solvent removed under reduced pressure. The crude product was purified by column chromatography on silica using ethyl acetate:hexane (7:3) as the mobile phase to afford 8-fluoro-4[5-(3-(morpholin-4-ylphenoxy)pyrimidin-2-yl]-3,4-dihydro-2*H*-1,4-benzo[1,4]oxazine (150mg, 27%) as white solid.
**Mass:** (ES+) 409$(M+H)^+$
**HPLC:** 98.5%
**NMR:** $\delta_H$ ($d_6$-DMSO) 3.08 (4H, m), 3.70 (4H, m), 4.20 (2H, m), 4.34 (2H, m), 6.38 (1H, dd), 6.63 (1H, br s), 6.70 (1H, dd), 6.81 (1H, m), 6.88 (1H, m), 7.18 (1H, t), 7.82 (1H, d) and 8.44 (2H, s).

**Example 2: 6-Fluoro-4-[5-(3-morpholinophenoxy)pyrimidin-2-yl]-3,4-dihydro-2H-benzo[1,4]oxazine**

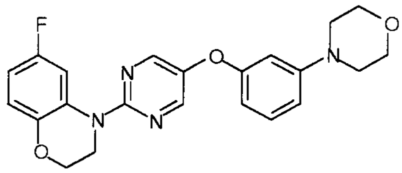

A suspension of 4-[3-(2-chloropyrimidin-5-yloxy)phenyl]morpholine (400mg, 1.37mmol), 6-fluoro-2*H*-1,4-benzoxazin-3(4*H*)-one (210 mg, 1.37mmol), *tris*(dibenzyl-ideneacetone)palladium(0) (62mg, 0.067mmol), 4,5-*bis*(diphenylphosphino)-9,9-dimethylxanthene (79mg, 0.137mmol) and cesium carbonate (895mg, 2.75mmol) in degassed 1,4-dioxane (8mL) was heated at 80°C for 2 days. The suspension was diluted with ethyl acetate and washed with water and brine. The organic phase was dried ($MgSO_4$) and the solvent removed under reduced pressure. The crude product was purified by column chromatography on silica using ethyl acetate:hexane (7:3) as the mobile phase to afford 6-fluoro-4-[5-(3-morpholinophenoxy)pyrimidin-2-yl]-3,4-dihydro-2H-benzo[1,4]oxazine (110mg, 20%) as white solid.
**Mass:** (ES+) 409$(M+H)^+$
**HPLC:** 97.1%
**NMR:** $\delta_H$ ($d_6$-DMSO) 3.08 (4H, m), 3.70 (4H, m), 4.15 (2H, m), 4.25 (2H, m), 6.38 (1H, dd), 6.64 (1H, br s), 6.70 (1H, dd), 6.78 (1H, m), 6.88 (1H, m), 7.18 (1H, t), 8.00 (1H, dd) and 8.48 (2H, s).

**Example 3: 6-Cyano-4-[5-(3-morpholinophenoxy)pyrimidin-2-yl]-3,4-dihydro-2H-benzo[1,4]oxazine**

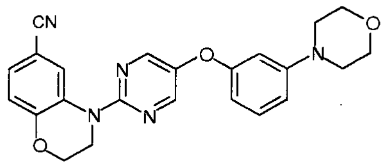

A suspension of 4-[3-(2-chloropyrimidin-5-yloxy)phenyl]morpholine (200mg, 0.685mmol), 6-cyano-2*H*-1,4-benzoxazin-3(4*H*)-one (110mg, 0.685mmol), *tris*(dibenzyl-ideneacetone) palladium(0) (31.9mg,0.034mmol), 4,5-*bis*(diphenylphosphino)-9,9-dimethylxanthene (40 mg, 0.068mmol) and cesium carbonate (448mg, 1.374mmol) in degassed 1,4-dioxane (5mL) was heated at 80°C for 2 days. The suspension was diluted with ethyl acetate and washed with water and brine. The organic phase was dried ($MgSO_4$) and the solvent removed under reduced pressure. The crude product was purified by column chromatography on silica using ethyl acetate:hexane (7:3) as the mobile phase to afford 6-cyano-4-[5-(3-morpholinophenoxy)pyrimidin-2-yl]-3,4-dihydro-2H-benzo[1,4]oxazine (75mg, 26%) as white solid.
**Mass:** (ES+) 416 $(M+H)^+$
**HPLC:** 99.3%
**NMR:** $\delta_H$ ($d_6$-DMSO) 3.10 (4H, m), 3.70 (4H, m), 4.20 (2H, m), 4.38 (2H, m), 6.38 (1H, dd), 6.64 (1H, br s), 6.70 (1H, dd), 7.06 (1H, d), 7.20 (1H, t), 7.39 (1H, d), 8.50 (1H) and 8.50 (2H, s).

**Example 4: 4-[5-(3-morpholinophenoxy)pyrimidin-2-yl]- 3,4-dihydro-2H-benzo-[1,4]oxazine**

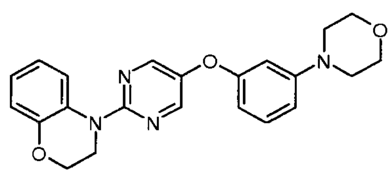

This compound was prepared by the same method as that used to prepare Example 1. **Mass:** (ES+) 391 $(M+H)^+$

**HPLC:** 97.4%

**NMR:** $\delta_H$ ($d_6$-DMSO) 3.08 (4H, m), 3.70 (4H, m), 4.17 (2H, m), 4.27 (2H, m), 6.36 (1H, dd), 6.62 (1H, br s), 6.68 (1H, dd), 6.85 (2H, m), 6.93 (1H, m), 7.17 (1H, t), 7.95 (1H, d) and 8.42 (2H, s).

**Example 5: 8-Fluoro-4-[5-[3-(4-methylpiperazin-1-yl)phenoxy]pyrimidin-2-yl]-3,4-dihydro-2H-benzo[1,4]oxazine**

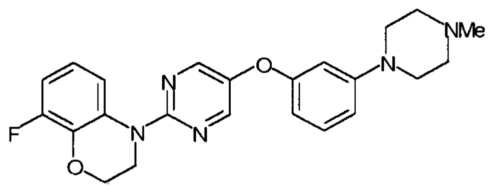

A suspension of 1-[3-(2-chloropyrimidin-5-yloxy)phenyl]-4-methylpiperazine (200mg, 0.656mmol), 8-fluoro-2*H*-1,4-benzoxazine (100mg, 0.656mmol), *tris*(dibenzylidene-acetone)palladium(0) (30mg, 0.032mmol), 4,5-*bis*(diphenylphosphino)-9,9-dimethylxanthene (38mg, 0.065mmol) and cesium carbonate (427mg, 1.31mmol) in degassed 1,4-dioxane (5mL) was heated at 80°C for 2 days. The suspension was diluted with ethyl acetate and washed with water and brine. The organic phase was dried ($MgSO_4$) and the solvent removed under reduced pressure. The crude product was purified by column purification on silica using dichloromethane:methanol (19:1) to afford 8-fluoro-4-[5-[3-(4-methylpiperazin-1-yl)phenoxy]pyrimidin-2-yl]-3,4-dihydro-2H-benzo[1,4]oxazine (38mg, 14%) as white solid.

**Mass:** (ES+) 422$(M+H)^+$

**HPLC:** 99.4%

**NMR:** $\delta_H$ ($d_6$-DMSO) 2.83 (3H, s), 2.95 (2H, br t), 3.10 (2H, m), 3.48 (2H, br d), 3.87 (2H, br d), 4.20 (2H, m), 4.34 (2H, m), 6.45 (1H, dd), 6.74 (1H, br s), 6.77 (1H, dd), 6.82 (1H, m), 6.89 (1H, m), 7.22 (1H, t), 7.81 (1H, d) and 8.44 (2H, s).

**Example 6: 8-Fluoro-4-[5-(3-morpholinophenoxy)-2-pyridyl]-3,4-dihydro-2H-benzo-[1,4]oxazine**

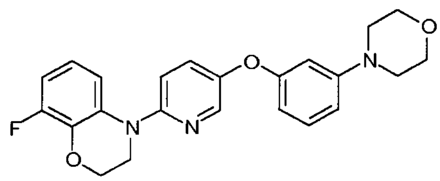

A suspension of 4-[3-(6-chloropyridin-3-yloxy)phenyl]morpholine (200mg, 0.689mmol), 8-fluoro-2*H*-1,4-benzoxazine (106 mg, 0.689mmol), *tris*(dibenzylideneacetone)-palladium(0) (32 mg, 0.034mmol), 4,5-*bis*(diphenylphosphino)-9,9-dimethyl-xanthene (40mg, 0.068mmol) and cesium carbonate (449mg, 1.38mol) in degassed 1,4-dioxane (5mL) was heated at 80°C for 2 days. The suspension was diluted with ethyl acetate and washed with water and brine. The organic phase was dried ($MgSO_4$) and the solvent removed under reduced pressure. The crude product was purified by column chromatography using ethyl acetate:hexane (7:3) as the mobile phase to afford 8-fluoro-4-[5-(3-morpholinophenoxy)-2-pyridyl]-2,3-dihydro-1,4-benzoxazine (100mg, 36%) as white solid.

**Mass:** (ES+) 408 $(M+H)^+$

**HPLC:** 99.1 %

**NMR:** $\delta_H$ ($d_6$-DMSO) 3.08 (4H, m), 3.70 (4H, m), 3.95 (2H, m), 4.29 (2H, m), 6.35 (1H, dd), 6.62 (1H, br s), 6.70 (1H, dd), 6.72-6.83 (2H, m), 7.12 (1H, m), 7.18 (1H, t), 7.24 (1H, d), 7.40 (1H, d) and 8.11 (1H, s).

**Example 7: 9-Fluoro-5-[5-(3-morpholin-4-ylphenoxy)pyrimidin-2-yl]-2,3,4,5-tetra-hydro-benzo[b][1,4]oxazepine**

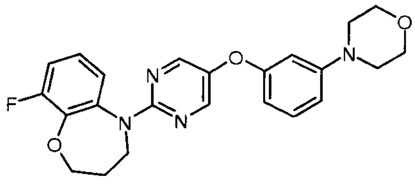

A suspension of 4-[3-(2-chloropyrimidin-5-yloxy)phenyl]morpholine (175mg, 0.6mmol), 9-fluoro-2,3,4,5-tetrahydrobenzo[b][1,4]oxazepine (100mg, 0.6mmol), *tris*(dibenzyl-ideneacetone)palladium(0) (27.5mg, 0.03mmol), 4,5-*bis*(diphenyl-phosphino)-9,9-dimethylxanthene (35mg, 0.06mmol) and cesium carbonate (392mg, 1.2mmol) in degassed 1,4-dioxane (8mL) was heated at 80°C for 2 days. The reaction mixture was diluted with ethyl acetate and washed with water and brine. The organic phase was dried ($MgSO_4$) and the solvent was removed under reduced pressure. The crude product was purified by column chromatography using ethyl acetate:hexane (7:3) as the mobile phase to afford 9-fluoro-5-[5-(3-morpholinophenoxy)pyrimidin-2-yl]-2,3,4,5-tetrahydrobenzo-[b][1,4]oxazepine (50mg, 20%) as a white solid.
**Mass:** (ES+) 423 $(M+H)^+$
**HPLC:** 99.5%
**NMR:** $\delta_H$($d_6$-DMSO) 2.05 (2H, m), 3.10 (4H, m), 3.72 (4H, m), 4.05 (2H, m), 4.16 (2H, m), 6.32 (1H, dd), 6.62 (1H, br s), 6.69 (1H, dd), 7.01-7.08 (1H, m), 7.10-7.22 (3H, m) and 8.30 (2H, s).

**Example 8: 1-[5-(3-Morpholin-4-ylphenoxy)pyrimidin-2-yl]-2,3-dihydro-1H-pyrido-[2,3-b][1,4]oxazine**

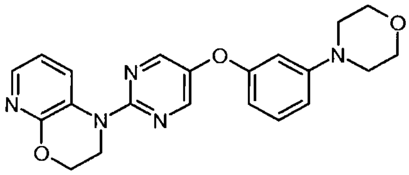

A suspension of 4-[3-(2-chloropyrimidin-5-yl)oxyphenyl]morpholine (214mg, 0.735mmol), 2,3-dihydro-1H-pyrido[2,3-b][1,4]oxazine (100mg, 0.735mmol), *tris*(dibenzylideneacetone)palladium(0) (33mg, 0.036mmol), 4,5-*bis* (diphenylphosphino)-9,9-dimethylxanthene (42.5mg, 0.073mmol) and cesium carbonate (478mg, 1.47mmol) in degassed 1,4-dioxane (8mL) was heated at 80°C for 2 days. The reaction mixture was diluted with ethyl acetate and washed with water and brine. The organic phase was dried ($MgSO_4$) and the solvent removed under reduced pressure. The crude product was purified by column chromatography using ethyl acetate:hexane (7:3) as the mobile phase to afford 1-[5-(3-morpholin-4-ylphenoxy)pyrimidin-2-yl]-2,3-dihydro-1H-pyrido[2,3-b][1,4]-oxazine (36mg, 12.5%) as a white solid.
**Mass:** (ES+) 392 $(M+H)^+$
**HPLC:** 100%
**NMR:** $\delta_H$ ($d_6$-DMSO) 3.11 (4H, m), 3.72 (4H, m), 4.20 (2H, m), 4.32 (2H, m), 6.40 (1H, dd), 6.64 (1H, br s), 6.72 (1H, dd), 6.99 (1H, m), 7.20 (1H, t), 7.85 (1H, dd), 8.50 (2H, s) and 8.52 (1H, m).

**Example 9: (R)-8-Fluoro-4-[5-(3-methylmorpholin-4-ylphenoxy)pyrimidin-2-yl]-3,4-dihydro-2H-benzo[1,4]oxazine**

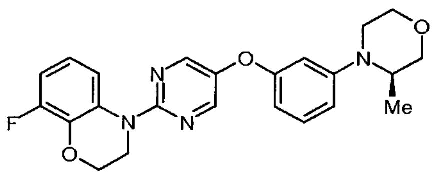

A suspension of (R)-4-[3-(2-chloropyrimidin-5-yloxy)phenyl]-3-methylmorpholine (255mg, 0.833mmol), 8-fluoro-3,4-dihydro-2H-benzo[1,4]oxazine (128mg, 0.833mmol), *tris*(dibenzylideneacetone)palladium(0) (38mg,

0.041mmol), 4,5-*bis*(diphenyl-phosphino)-9,9-dimethylxanthene (48 mg, 0.083mmol) and cesium carbonate (543mg, 1.66 mol) in de-gassed 1,4-dioxane (5mL) was heated at 80°C for 2 days. The reaction mixture was diluted with dichloromethane and washed with water and brine. The organic phase was dried ($MgSO_4$) and the solvent removed under reduced pressure. The crude product was purified by flash chromatography, using 0-15% ethyl acetate:hexane as eluent, and further purified by preparative HPLC to afford (R)-8-fluoro-4-[5-(3-methylmorpholin-4-ylphenoxy)pyrimidin-2-yl]-3,4-dihydro-2H-benzo[1,4]oxazine (23mg, 6.5%) as a white solid.
Mass: (ES+) 422$(M+H)^+$
Chiral HPLC: 99.8%
NMR: $\delta_H$ ($d_6$-DMSO) 2.97 (2H, m), 3.20 (1H, m), 3.52 (2H, m), 3.66 (3H, d), 3.84-3.94 (2H, m), 4.22 (2H, m), 4.36 (2H, m), 6.34 (1H, dd), 6.59 (1H, br s), 6.67 (1H, dd), 6.83 (1H, m), 6.91 (1H, m), 7.19 (1H, t), 7.82 (1H, d) and 8.45 (2H, s).

**Example 10: (S)-8-Fluoro-4-[5-(3-methylmorpholin-4-ylphenoxy)pyrimidin-2-yl]-3,4-dihydro-2H-benzo[1,4]oxazine**

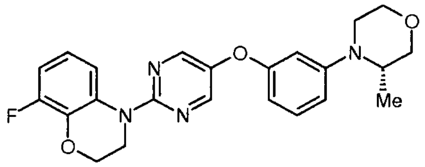

A suspension of (S)-4-[3-(2-chloropyrimidin-5-yloxy)phenyl]-3-methylmorpholine (176mg, 0.575mmol), 8-fluoro-3,4-dihydro-2H-benzo[1,4]oxazine (88mg, 0.575mmol), *tris*(dibenzylideneacetone)palladium(0) (26mg, 0.028mmol), 4,5-*bis*(diphenyl-phosphino)-9,9-dimethylxanthene (33mg, 0.058mmol) and cesium carbonate (374 mg, 1.15mmol) in de-gassed 1,4-dioxane (5mL) was heated at 80°C for 2 days. The suspension was diluted with dichloromethane and washed with water and brine. The organic phase was dried ($MgSO_4$) and the solvent removed under reduced pressure. The crude product was purified initially by flash chromatography, using 0-15% ethyl acetate:hexane as eluent, and further purified by preparative chiral HPLC to give (S)-8-fluoro-4-[5-(3-methylmorpholin-4-ylphenoxy)-pyrimidin-2-yl]-3,4-dihydro-2H-benzo-[1,4]oxazine (33mg, 14%) as a white solid.
**Mass:** (ES+) 422$(M+H)^+$
**HPLC:** 100.0%
**NMR:** $\delta_H$ ($d_6$-DMSO) 2.97 (2H, m), 3.20 (1H, m), 3.52 (2H, m), 3.66 (3H, d), 3.84-3.94 (2H, m), 4.22 (2H, m), 4.36 (2H, m), 6.34 (1H, dd), 6.59 (1H, br s), 6.67 (1H, dd), 6.83 (1H, m), 6.91 (1H, m), 7.19 (1H, t), 7.82 (1H, d) and 8.45 (2H, s).

**Example 11: 8-Fluoro-4-[5-(3-methoxyphenoxy)pyrimidin-2-yl]-3,4-dihydro-2H-benzo[1,4]oxazine**

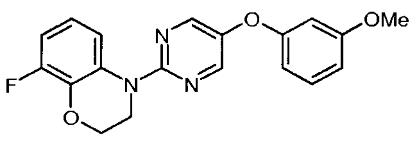

A suspension of 2-chloro-5-(3-methoxyphenoxy)pyrimidine (200mg, 0.847mmol), 8-fluoro-3,4-dihydro-2H-benzo[1,4]oxazine (130mg, 0.847mmol), *tris*(dibenzylidene-acetone)palladium(0) (39mg, 0.042mmol), 4,5-*bis*(diphenylphosphino)-9,9-dimethylxanthene (49mg, 0.084mmol) and cesium carbonate (552mg, 1.69mmol) in de-gassed 1,4-dioxane (8mL) was heated at 80°C for 2 days. The reaction mixture was diluted with dichloromethane and washed with water and brine. The organic phase was dried ($MgSO_4$), the solvent removed under reduced pressure and the crude product was purified by flash chromatography, using 0-15% ethyl acetate:hexane as eluent, to provide 8-fluoro-4-[5-(3-methoxyphenoxy)pyrimidin-2-yl]-3,4-dihydro-2H-benzo[1,4]oxazine (110mg, 37%) as a white solid.
Mass: (ES+) 354$(M+H)^+$
HPLC: 99.5%
NMR: $\delta_H$ ($d_6$-DMSO) 3.74 (3H, s), 4.22 (2H, m), 4.36 (2H, m), 6.57 (1H, dd), 6.64 (1H, br s), 6.71 (1H, dd), 6.83 (1H, m), 6.91 (1H, m), 7.27 (1H, t), 7.83 (1H, d) and 8.48 (2H, s).

**Example 12: 8-Fluoro-4-[5-(3-N,N-dimethylaminophenoxy)pyrimidin-2-yl]-3,4-dihydro-2H-benzo[1,4]oxazine**

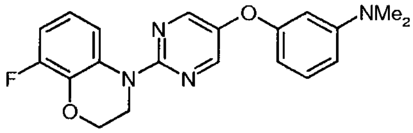

A suspension of 2-chloro-5-(3-N,N-dimethylaminophenyl)pyrimidine (210mg, 0.000847mol), 8-fluoro-3,4-dihydro-2H-benzo[b][1,4]oxazine (130mg, 0.000847mol), *tris*(dibenzylideneacetone)palladium(0) (39mg, 0.000042mol), 4,5-*bis*(diphenylphosphino)-9,9-dimethylxanthene (49 mg, 0.000084mol) and cesium carbonate (552mg, 0.00169mol) in de-gassed 1,4-dioxane (8mL) was heated at 80°C for 2 days. The suspension was diluted with dichloromethane and washed with water and brine. The organic phase was dried ($MgSO_4$) and the solvent removed under reduced pressure. The crude product was purified by flash chromatography using 0-5% ethyl acetate:hexane as eluent to obtained 8-fluoro-4-[5-(3-N,N-dimethylaminophenoxy)pyrimidin-2-yl]-3,4-dihydro-2H-benzo[1,4]oxazine (110mg, 37%) as white solid.
Mass: (ES+) $367(M+H)^+$
HPLC: 99.8%
NMR: $\delta_H$ ($d_6$-DMSO) 2.88 (3H, s), 4.20 (2H, m), 4.35 (2H, m), 6.21 (1H, dd), 6.40 (1H, br s), 6.49 (1H, dd), 6.82 (1H, m), 6.91 (1H, m), 7.15 (1H, t), 7.82 (1H, d) and 8.45 (2H, s).

**Example 13: 4-(5-(3-Bromophenoxy)pyrimidine-2yl)-8-fluoro-3,4-dihydro-2H-benzo[1,4]oxazine**

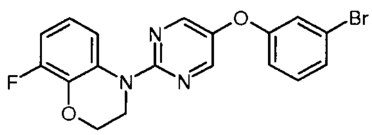

A mixture of 8-fluoro-4-[5-(hydroxy)pyrimidin-2yl]-3,4-dihydro-2H-benzo-[1,4]oxazine (0.818g, 0.0033mol), 3-bromophenylboronic acid (0.797g, 0.0039mol), copper (II) acetate (0.6 g, 0.0029mol), triethylamine (2.3mL, 0.0165mol) and powdered 4Å molecular sieves in dichloromethane (40mL) was stirred under air for 3 days. A calcium chloride guard tube was used to protect the reaction from moisture. The reaction mixture was diluted with dichloromethane, filtered and washed with water and brine. The organic phase was dried ($MgSO_4$) and the solvent removed under reduced pressure. The crude product was purified by flash chromatography, using 0-15% ethyl acetate:hexane as eluent, to give 4-(5-(3-bromo-phenoxy)pyrimidine-2yl)-8-fluoro-3,4-dihydro-2H-benzo-[1,4]oxazine (0.614g, 61%) as a white solid.
**Mass:** (ES+) 403 $(M+H)^+$
**HPLC:** 96.2%
**NMR:** $\delta_H$ ($d_6$-DMSO) 4.22 (2H, m), 4.36 (2H, m), 6.84 (1H, m), 6.92 (1H, m), 7.08 (1H, m), 7.28 (1H, m), 7.30-7.38 (2H, m), 7.83 (1H, br d) and 8.53 (2H, s).

**Example 14: 1-{3-[2-(8-Fluoro-2,3-dihydro-benzo[1,4]oxazin-4-yl)-pyrimidin-5-yloxy]-phenyl}azetidin-3-ol**

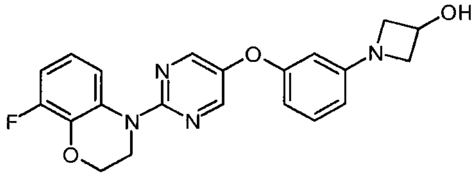

A suspension of 4-(5-(3-bromo-phenoxy)pyrimidine-2yl)-8-fluoro-3,4-dihydro-2H-benzo-[1,4]oxazine (300 mg, 0.746mmol), azetidin-3-ol hydrochloride (98mg, 0.895mmol), *tris*(dibenzylideneacetone)palladium(0) (34mg,

0.037mmol), 4,5-*bis*(diphenyl-phosphino)-9,9- dimethylxanthene (43 mg, 0.074mmol) and cesium carbonate (324 mg, 0.99mmol) in de-gassed 1,4-dioxane (5mL) was heated at 80°C for 2 days. The reaction mixture was diluted with dichloromethane and washed with water and brine. The organic phase was dried ($MgSO_4$) and the solvent removed under reduced pressure. The crude product was purified by flash chromatography, using 0-15% ethyl acetate:hexane as eluent, to give 1-{3-[2-(8-fluoro-2,3-dihydrobenzo[1,4]oxazin-4-yl)-pyrimidin-5-yloxy]-phenyl}azetidin-3-ol (110mg, 56%) as a white solid.

**Mass:** (ES+) $395(M+H)^+$

**HPLC:** 99.8%

**NMR:** $\delta_H$ ($d_6$-DMSO) 3.50 (2H, m), 4.05 (2H, t), 4.21 (2H, m), 4.36 (2H, m), 4.55 (1H, m), 5,62 (1H, d), 6.10 (1H, br s), 6.19 (1H, dd), 6.25 (1H, dd), 6.83 (1H, m), 6.91 (1H, m), 7.14 (1H, t), 7.83 (1H, br d) and 8.44 (2H, s).

**Example 15: 4-[5-(3-Morpholin-4-yl-phenoxy)pyridin-2-yl]-3,4-dihydro-2H-benzo[1,4]oxazine-6-carbonitrile**

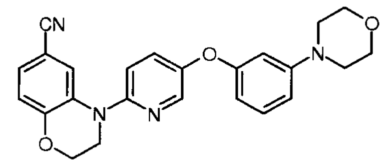

A suspension of 4-{3-[(6-chloropyridin-3-yl)oxy]phenyl}morpholine (200mg, 0.689mmol), 3,4-dihydro-2H-benzo[1,4]oxazine-6-carbonitrile (110mg, 0.689 mol), *tris*(dibenzylideneacetone)palladium(0) (31mg, 0.034mmol), 4,5-*bis*(diphenylphosphino)-9,9-dimethylxanthene (30 mg, 0.068mmol) and cesium carbonate (449mg, 1.379mmol) in de-gassed 1,4-dioxane (5mL) was heated at 80°C for 2 days. The reaction mixture was diluted with ethyl acetate and washed with water and brine. The organic phase was dried ($MgSO_4$) and the solvent removed under reduced pressure. The crude product was purified by column chromatography using ethyl acetate:hexane (7:3) as the mobile phase to afford 4-[5-(3-Morpholin-4-yl-phenoxy)pyridin-2-yl]-3,4-dihydro-2H-benzo[1,4]oxazine-6-carbonitrile (110mg, 39%) as a white solid.

Mass: (ES+) $415(M+H)^+$

HPLC: 98.7%

NMR: $\delta_H$ ($d_6$-DMSO) 3.12 (4H, m), 3.72 (4H, m), 3.94 (2H, m), 4.34 (2H, m), 6.40 (1H, dd), 6.55 (1H, br s), 6.74 (1H, dd), 7.04 (1H, d), 7.22 (1H, t), 7.30 (2H, m), 7.47 (1H, dd), 7.82 (1H, d) and 8.65 (1H, d).

**Example 16: 8-Fluoro-3-methyl-4-[5-(3-morpholin-4-yl-phenoxy)pyrimidin-2-yl]-3,4-dihydro-2H-benzo[1,4]oxazine**

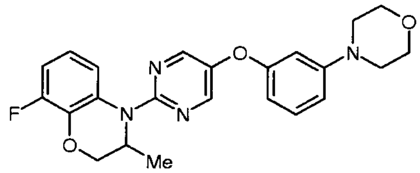

A suspension of 4-[3-(2-chloropyrimidin-5-yl)oxy)phenyl]morpholine (611mg, 0.0021mol), (±)-8-fluoro-3-methyl-3,4-dihydro-2H-benzo[1,4]oxazine (350mg, 0.0021mol), *tris*(dibenzylideneacetone)palladium(0) (96mg, 0.000105mol), 4,5-*bis*(diphenylphosphino)-9,9-dimethylxanthene (121mg, 0.00021mol) and cesium carbonate (1.36g, 0.0042mol) in de-gassed 1,4-dioxane (10mL) was heated at 90°C for 24h. The reaction mixture was diluted with ethyl acetate and washed with water and brine. The organic phase was dried ($MgSO_4$) and the solvent removed under reduced pressure. The crude product was purified by column chromatography using ethyl acetate: hexane (7:3) as a mobile phase to afford 8-Fluoro-3-methyl-4-[5-(3-morpholin-4-yl-phenoxy)pyrimidin-2-yl]-3,4-dihydro-2H-benzo[1,4]oxazine (280 mg) as a yellow solid. The enantiomers were separated by chiral prep HPLC giving 75mg of each isomer.

Mass: (ES+) 423 $(M+H)^+$

HPLC: Fraction-1 100% (chiral), Fraction-2 98.1% (chiral)

NMR: $\delta_H$ ($d_6$-DMSO) 1.25 (3H, d), 3.11 (4H, m), 3.70 (4H, m), 4.14 (1H, br d), 4.38 (1H, d), 5.17 (1H, m), 6.40 (1H, dd),

6.67 (1H, br s), 6.72 (1H, dd), 6.78-6.92 (2H, m), 7.20 (1H, t), 7.85 (1H, d) and 8.45 (2H, s).

**Example 17: Preparation of stock solutions for biological assays**

Aβ(1-42) preparation

Aβ(1-42) was prepared for amyloid aggregation and toxicity assays by dissolving Aβ(1-42) HCl salt in hexafluoroisopropanol (HFIP), with brief sonication and vortexing. This solution of the Aβ(1-42) peptide in HFIP was stored at 4°C @ 2mM. When required, an aliquot of this stock solution was freeze-dried and dissolved in DMSO to 200 times the required final assay concentration (e.g. 2mM for a final assay concentration of 10 μM).

Compound preparation

A 20mM stock solution of each test compound was prepared in DMSO, and aliquots of these solutions were used to prepare further stock solutions of each test compound in DMSO, ranging in concentration from 3μM up to 10mM. These stock solutions were prepared for use as and when required and stored at -20°C (maximum of 3 freeze-thaw cycles). The 20mM parent stock solutions were stored frozen at -20 °C.

**Example 18: Cell viability assay for amyloid toxicity using MTT reduction**

The activity of compounds in protecting SH SY5Y cells from a toxic insult of 10μM Aβ(1-42) was assessed by using inhibition of MTT reduction as a measure of cell viability. An aliquot (3μl) of test compound [various concentrations] in DMSO is added to 294 μl of Opti-Mem (containing 2% FBS, 1% Pen/Strep, 1% L-Gln) {daughter plate}. The well is mixed thoroughly. Then an aliquot (3 μl) of Aβ(1-42) [2mM] is added to the daughter plate wells and again mixed thoroughly. 50 μl is then aspirated and dispensed into wells containing 50 μl media + SH SY5Ycells (cells are also plated in Opti-Mem, at ~ 30,000 cells/well/50 μl). Final concentrations of compound on cells range from [50μM] to [~15nM] with a final concentration of Aβ(1-42) of [10μM].

Cell plates are incubated for 24 h and then the MTT assay (Shearman, 1999), is performed. Briefly, 15μl of MTT (3-(4,5-dimethylthiazol-2-yl)-2,5-diphenyltetrazolium bromide) dye (from Promega) added to each well and the plates incubated in 5% $CO_2$ at 37°C for 4 hours. 100 μl Stop/solubilsation solution (from Promega) was added to each well and the plates were left overnight in humidified box at room temperature. The plate was shaken and the absorbance was recorded at both 570 nm and 650 nm. ΔA values were calculated by subtracting absorbance at 650 nm from absorbance at 570 nm, to reduce non-specific background absorbance. ΔA values from equivalent experiments were averaged and % cell viability was determined as follows:

$$\%\ \text{cell viability} = \frac{[\Delta A(\text{sample}) - \Delta A(\text{dead cell control})] \times 100\%}{[\Delta A(\text{live cell control}) - \Delta A(\text{dead cell control})]}$$

Live cell controls: 1% DMSO in Opti-Mem
Dead cell controls: 0.1% Triton X-100 added to cells

**Example 19: Activity of compounds in protecting SH SY5Y cells from a toxic insult of 10μM Aβ(1-42) using inhibition of MTT reduction as a measure of cell viability**

| | $IC_{50}$ (μM) |
|---|---|
| Example 1 | 23 |
| Example 2 | 26 |
| RS-0406 | 35 |

**Example 20**

Long-term potentiation (LTP) is a natural, prolonged electrophysiological response associated with the neurological processes of learning and short-term memory, which are affected in Alzheimer's disease. Aβ(1-42), has been shown to act as a potent inhibitor of LTP, and as a result LTP has been used as a model system for testing the efficacy of potential therapeutic agents for the disease (Walsh et al. 2002; Rowan et al. 2004). LTP was measured using stimulation of Schaffer collaterals in rat hippocampal brain slices, as described below.

Sample preparation

20μL of 5mM Example 1 in DMSO was added to the stock Aβ(1-42) (1mL) and agitated at 37°C for 24hr. On the experimental day the mixture is diluted to 33mL in aCSF (32mL) immediately prior to use. The final concentration of Example 1 was 3μM. The final concentration of Aβ(1-42) was 1μM and the final concentration of DMSO was 0.06%

Preparation and pre-treatment of brain slices

Male young adult Sprague Dawley rats (6-8 weeks, 200 - 250g) were humanely killed by cervical dislocation. Transverse hippocampal slices of 400 μm thickness were cut in artificial cerebrospinal fluid (aCSF: 127 mM NaCl, 1.6 mM KCl, 1.24 mM $KH_2PO_4$, 1.3 mM $MgSO_4$, 2.4 mM $CaCl_2$, 26 mM $NaHCO_3$, 10 mM glucose) at below 4 °C using a microslicer (Leica VT1000S). Slices were subsequently maintained in oxygenated aCSF (95% $O_2$, 5% $CO_2$) at room temperature for at least 1 hr before electrophysiological recordings. After this recovery period, the slices were transferred to a modified 'Haas' chamber and constantly perfused with warmed (~30°C) aerated aCSF at a rate of 1.5-3.0 mL/min.

Electrophysiological recordings

Schaffer collaterals were stimulated (3-22V, 0.1ms pulse width, 0.03Hz) with a concentric bipolar electrode and the evoked extracellular field excitatory postsynaptic potentials (fEPSPs) were recorded via an Axoclamp 2A amplifier from the *stratum radiatum* of the CA1 region of the hippocampus with a glass capillary microelectrode filled with 4M NaCl (resistance 2-4 MΩ). Stimulation parameters were set to produce a fEPSP of approximately 50% of the maximum amplitude. A 10 minute stable baseline period (control) was recorded followed by administration of test compound 10 minutes prior to high frequency stimulation (1sec 100Hz train) applied three times at 10 minute intervals with an 80 minute wash of test compound after the final high frequency stimulation protocol.

The results obtained for Example 1 (Figures 1- 3) indicate that the toxic effect of 1μM Aβ(1-42) on LTP is effectively blocked by this compound at a concentration of 3μM. Legends for Figures:

Figure 1: The effects of Example 1 (3μM) on Aβ(1-42)-induced depression of LTP

Figure 2: Histogram summary for the effects of Example 1 (3μM) on Aβ(1-42)-induced depression of LTP

Figure 3: Tabulated summary for the effects of Example 1 (3μM) on Aβ(1-42)-induced depression of LTP

Statistical analysis: data are presented as a mean S.E.M. Significant differences of data were calculated using the Students t-test. Probability values of $P < 0.05$ are considered to represent significant differences.

**REFERENCES**

Buxbaum, J. N. (2004). The systemic amyloidoses. Curr Opin Rheumatol 16, 67-75.

Caughey, B. and P. T. Lansbury (2003). Protofibrils, pores, fibrils, and neurodegeneration: separating the responsible protein aggregates from the innocent bystanders. Annu Rev Neurosci 26, 267-298.

Cleary, J. P., D. M. Walsh, J. J. Hofmeister, G. M. Shankar, M. A. Kuskowski, D. J. Selkoe and K. H. Ashe (2005). Natural oligomers of the amyloid-β protein specifically disrupt cognitive function. Nat Neurosci 8, 79-84.

Dev, K. K., K. Hofele, S. Barbieri, V. L. Buchman and H. van der Putten (2003). Part II: alpha-synuclein and its molecular pathophysiological role in neurodegenerative disease. Neuropharmacology 45, 14-44.

Estrada, L. D. and C. Soto (2007). Disrupting β-amyloid aggregation for Alzheimer's disease treatment. Curr Topics Med Chem 7, 115-126.

Forman, M. S., J. Q. Trojanowski and V. M. Lee (2004). Neurodegenerative diseases: a decade of discoveries paves the way for therapeutic breakthroughs. Nat Med 10, 1055-1063.

Gejyo, F., T. Yamada, S. Odani, Y. Nakagawa, M. Arakawa, T. Kunitomo, H. Kataoka, M. Suzuki, Y. Hirasawa, T. Shirahama and et al. (1985). A new form of amyloid protein associated with chronic hemodialysis was identified as beta 2-microglobulin. Biochem Biophys Res Commun 129, 701-706.

Glabe, C. G. (2004). Conformation-dependent antibodies target diseases of protein misfolding. Trends Biochem Sci 29, 542-547.

Glenner, G. G. (1980a). Amyloid deposits and amyloidosis: the beta-fibrilloses. N Engl J Med 302, 1283-1292.

Glenner, G. G. (1980b). Amyloid deposits and amyloidosis: the beta-fibrilloses. N Engl J Med 302, 1333-1343.

Haataja, L., T. Gurlo, C. J. Huang and P. C. Butler (2008). Islet amyloid in type 2 diabetes, and the toxic oligomer hypothesis. Endocrine Rev 29, 303-316.

Jaikaran, E. T. and A. Clark (2001). Islet amyloid and type 2 diabetes: from molecular misfolding to islet pathophysiology. Biochim Biophys Acta 1537, 179-203.

Kagan, B. L., Y. Hirakura, R. Azimov, R. Azimova and M. C. Lin (2002). The channel hypothesis of Alzheimer's disease: current status. Peptides 23, 1311-1315.

Kayed, R., E. Head, J. L. Thompson, T. M. McIntire, S. C. Milton, C. W. Cotman and C. G. Glabe (2003). Common structure of soluble amyloid oligomers implies common mechanism of pathogenesis. Science 300, 486-489.

LeVine, H., III (2004). The amyloid hypothesis and the clearance and degradation of Alzheimer's beta-peptide. J Alzheimers Dis 6, 303-314.

Lue, L. F., Y. M. Kuo, A. E. Roher, L. Brachova, Y. Shen, L. Sue, T. Beach, J. H.

Kurth, R. E. Rydel and J. Rogers (1999). Soluble amyloid beta peptide concentration as a predictor of synaptic change in Alzheimer's disease. Am J Pathol 155, 853-862.

McLean, C. A., R. A. Cherny, F. W. Fraser, S. J. Fuller, M. J. Smith, K. Beyreuther, A. I. Bush and C. L. Masters (1999). Soluble pool of Abeta amyloid as a determinant of severity of neurodegeneration in Alzheimer's disease. Ann Neurol 46, 860-866. Masino, L. (2004). Polyglutamine and neurodegeneration: structural aspects. Protein Pept Lett 11, 239-248.

Melnikova, I. (2007). Therapies for Alzheimer's disease. Nat Rev Drug Disc 6, 341-342.

Ross, C. A. and M. A. Poirier (2004). Protein aggregation and neurodegenerative disease. Nat Med 10 Suppl: S10-7.

Selkoe, D. J. (2003). Folding proteins in fatal ways. Nature 426, 900-904.

Shah, R. S., H. G. Lee, Z. Xiongwei, G. Perry, M. A. Smith and R. J. Castellani (2008). Current approaches in the treatment of Alzheimer's disease. Biomed Pharmacother 62, 199-207.

Shearman, M. S. (1999). Toxicity of protein aggregates in PC12 cells: 3-(4,5-dimethylthiazol-2-yl)-2,5-diphenyltetrazolium bromide assay. Methods Enzymol 309, 716-723.

Soto, C. and J. Castilla (2004). The controversial protein-only hypothesis of prion propagation. Nat Med 10 Suppl: S63-7.

Stefani, M. (2004). Protein misfolding and aggregation: new examples in medicine and biology of the dark side of the protein world. Biochim Biophys Acta 1739, 5-25.

Taylor, J. P., J. Hardy and K. H. Fischbeck (2002). Toxic proteins in neurodegenerative disease. Science 296,

1991-1995.

Walsh, D. M., I. Klyubin, J. V. Fadeeva, W. K. Cullen, R. Anwyl, M. S. Wolfe, M. J. Rowan and D. J. Selkoe (2002). Naturally secreted oligomers of amyloid beta protein potently inhibit hippocampal long-term potentiation in vivo. Nature 416, 535-539.

Walsh, D. M. and D. J. Selkoe (2004). Oligomers on the brain: the emerging role of soluble protein aggregates in neurodegeneration. Protein Pept Lett 11, 213-228.

Walsh, D. M. and D. J. Selkoe (2007). Aβ oligomers - a decade of discovery, J Neurochem 101, 1172-1184.

Wang. J., D. W. Dickson, J. Q. Trojanowski and V. M. Lee (1999). The levels of soluble versus insoluble brain Abeta distinguish Alzheimer's disease from normal and pathologic aging. Exp Neurol 158, 328-337.

Wilcock, G. K., (2008). The pharmacological treatment of Alzheimer's disease with cholinesterase inhibitors and memantine. In Pharmacological Mechanisms in Alzheimer's Therapeutics, Cuello AC, ed. Springer, New York, pp. 36-49.

Wood, J. D., T. P. Beaujeux and P. J. Shaw (2003). Protein aggregation in motor neurone disorders. Neuropathol Appl Neurobiol 29, 529-545.

## Claims

1. A compound of formula (I) or a pharmaceutically acceptable salt thereof:

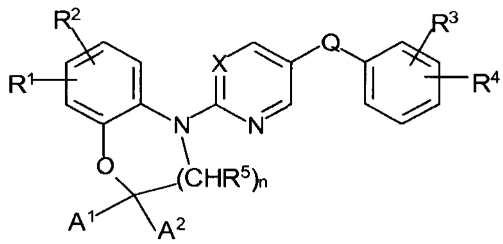

(I)

wherein

X is N or CH;
Q is $NR^6$ or O;
$A^1$ and $A^2$ are independently hydrogen or $C_{1-6}$ alkyl or may together form a carbonyl group;
$R^1$ and $R^2$ are independently hydrogen, halogen, $CF_3$, CN, $OR^7$, $OR^8$, $NR^8R^9$, $NR^8COR^{10}$, $NR^8SO_2R^{10}$, $SO_2NR^8R^9$, $SO_2R^{10}$ or $C_{1-6}$ alkyl optionally and independently substituted by one or more of hydroxyl, $C_{1-6}$ alkoxy, halogen or $NR^8R^9$;
$R^3$ is hydrogen, halogen, $CF_3$ or $OR^7$;
$R^4$ is hydrogen, halogen, $CF_3$, $OR^8$, $NR^8R^9$, $NR^8COR^{10}$, $NR^8SO_2R^{10}$ or $C_{1-6}$ alkyl optionally substituted by hydroxyl, $C_{1-6}$ alkoxy or $NR^8R^9$;
or when $R^3$ and $R^4$ are positioned ortho and taken together form $-O(CH_2)_mO-$, where m is 1-3;
$R^5$ is hydrogen or $C_{1-6}$ alkyl optionally substituted by hydroxyl, $C_{1-6}$ alkoxy or $NR^8R^9$;
$R^6$ is hydrogen or $C_{1-6}$ alkyl;
$R^7$ is hydrogen or $C_{1-6}$ alkyl optionally substituted by $OR^8$ or $NR^8R^9$;
$R^8$ is hydrogen, $C_{1-6}$ alkyl, optionally substituted by hydroxyl or $C_{1-6}$ alkoxy or $C_{1-3}$ alkylphenyl wherein said phenyl group is optionally substituted by one or more substituents selected from halogen, $C_{1-6}$ alkyl, $CF_3$, $OR^7$, $NR^8R^9$ or $OCF_3$;
$R^9$ is hydrogen, $C_{1-6}$ alkyl, $C_{1-6}$ alkenyl, $C_{1-6}$ alkynyl, phenyl or $C_{1-3}$ alkylphenyl wherein said phenyl groups are optionally substituted by one or more substituents selected from halogen, $C_{1-6}$ alkyl, $CF_3$, $OR^7$ or $OCF_3$;
or the groups $R^8$ and $R^9$ when they are attached to a nitrogen atom may together form a 5- or 6-membered ring

which optionally contains one further heteroatom selected from $NR^7$, S and O said 5 or 6 membered ring being optionally substituted by hydroxyl or $C_{1-6}$ alkoxy;

or the groups $R^8$ and $R^9$ when they are attached to a nitrogen atom may together form an azetidinyl ring optionally substituted by hydroxyl or $C_{1-6}$ alkoxy; and

$R^{10}$ is $C_{1-6}$ alkyl or a phenyl group optionally substituted by one or more substituents selected from halogen, $C_{1-6}$ alkyl, $CF_3$, $OCF_3$ or $OR^7$; and

n is 1 or 2.

2. A compound of formula (Ia) or a pharmaceutically acceptable salt thereof:

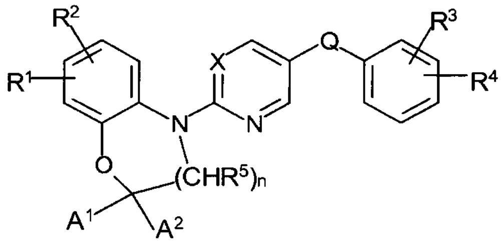

(Ia)

wherein

X is N or CH;

Q is $NR^6$ or O;

$A^1$ and $A^2$ are independently hydrogen or $C_{1-6}$ alkyl and may together form a carbonyl group;

$R^1$ and $R^2$ are independently hydrogen, halogen, $CF_3$, CN, $OR^7$, $OR^8$, $NR^8R^9$, $NR^8COR^{10}$, $NR^8SO_2R^{10}$, $SO_2NR^8R^9$, $SO_2R^{10}$ or $C_{1-6}$ alkyl optionally and independently substituted by one or more of hydroxyl, $C_{1-6}$ alkoxy, halogen or $NR^8R^9$;

$R^3$ is hydrogen, halogen, $CF_3$ or $OR^7$;

$R^4$ is hydrogen, halogen, $CF_3$, $OR^8$, $NR^8R^9$, $NR^8COR^{10}$, $NR^8SO_2R^{10}$ or $C_{1-6}$ alkyl optionally substituted by hydroxyl, $C_{1-6}$ alkoxy or $NR^8R^9$;

or when $R^3$ and $R^4$ are positioned ortho and taken together form $-O(CH_2)_mO-$, where m is 1-3;

$R^5$ is hydrogen or $C_{1-6}$ alkyl optionally substituted by hydroxyl, $C_{1-6}$ alkoxy or $NR^8R^9$;

$R^6$ is hydrogen or $C_{1-6}$ alkyl;

$R^7$ is hydrogen or $C_{1-6}$ alkyl optionally substituted by $OR^8$ or $NR^8R^9$;

$R^8$ is hydrogen, $C_{1-6}$ alkyl or $C_{1-3}$ alkylphenyl wherein said phenyl group is optionally substituted by one or more substituents selected from halogen, $C_{1-6}$ alkyl, $CF_3$, $OR^7$, $NR^8R^9$ or $OCF_3$;

$R^9$ is hydrogen, $C_{1-6}$ alkyl, $C_{1-6}$ alkenyl, phenyl or $C_{1-3}$ alkylphenyl wherein said phenyl groups are optionally substituted by one or more substituents selected from halogen, $C_{1-6}$ alkyl, $CF_3$, $OR^7$ or $OCF_3$;

or the groups $R^8$ and $R^9$ when they are attached to a nitrogen atom may together form a 5- or 6-membered ring which optionally contains one further heteroatom selected from $NR^7$, S and O; and

$R^{10}$ is $C_{1-6}$ alkyl or a phenyl group optionally substituted by one or more substituents selected from halogen, $C_{1-6}$ alkyl, $CF_3$, $OCF_3$ or $OR^7$; and

n is 1 or 2.

3. A compound as claimed in claim 1 or claim 2 wherein

X is N or CH;

Q is $NR^6$ or O;

$A^1$ and $A^2$ are independently hydrogen or $C_{1-6}$ alkyl and may together form a carbonyl group;

m = 1-2 and n = 1;

$R^1$ and $R^2$ are independently hydrogen, halogen, $CF_3$, CN, $OR^7$, $NR^8R^9$, $NR^8COR^{10}$, $NR^8SO_2R^{10}$ or $C_{1-6}$ alkyl optionally substituted by hydroxyl, $C_{1-6}$ alkoxy or $NR^8R^9$;

$R^3$ is hydrogen, halogen, $CF_3$ or $OR^7$;

$R^4$ is hydrogen, halogen, $CF_3$, $OR^8$, $NR^8R^9$, $NR^8COR^{10}$ or $NR^8SO_2R^{10}$;

$R^5$ is hydrogen or $C_{1-6}$ alkyl optionally substituted by hydroxyl, $C_{1-6}$ alkoxy or $NR^8R^9$;

$R^6$ is hydrogen or $C_{1-6}$ alkyl;

$R^7$ is hydrogen or $C_{1-6}$ alkyl optionally substituted by $OR^8$ or $NR^8R^9$;
$R^8$ is hydrogen, $C_{1-6}$ alkyl or $C_{1-3}$ alkylphenyl wherein said phenyl group is optionally substituted by one or more substituents selected from halogen, $C_{1-6}$ alkyl, $CF_3$, $OR^7$, $NR^8R^9$ or $OCF_3$;
$R^9$ is hydrogen, $C_{1-6}$ alkyl, $C_{1-6}$ alkenyl, phenyl or $C_{1-3}$ alkylphenyl wherein said phenyl groups are optionally substituted by one or more substituents selected from halogen, $C_{1-6}$ alkyl, $CF_3$, $OR^7$ or $OCF_3$;
or the groups $R^8$ and $R^9$ when they are attached to a nitrogen atom may together form a 5- or 6-membered ring which optionally contains one further heteroatom selected from $NR^7$, S and O and/or is optionally substituted by hydroxyl or $C_{1-6}$ alkoxy;
or the groups $R^8$ and $R^9$ when they are attached to a nitrogen atom may together form an azetidinyl ring optionally substituted by hydroxyl or $C_{1-6}$ alkoxy; and
$R^{10}$ is $C_{1-6}$ alkyl or a phenyl group optionally substituted by one or more substituents selected from halogen, $C_{1-6}$ alkyl, $CF_3$, $OCF_3$ or $OR^7$.

**4.** A compound as claimed in any one of claims 1 to 3, wherein

X is N or CH;
Q is $NR^6$ or O;
$A^1$ and $A^2$ are independently hydrogen or $C_{1-6}$ alkyl and may together form a carbonyl group;
m=1-2andn=1;
$R^1$ and $R^2$ are independently hydrogen, halogen, $CF_3$, CN, $OR^7$ or $NR^8R^9$;
$R^3$ is hydrogen, F, or $OR^7$;
$R^4$ is hydrogen, halogen, $CF_3$, $OR^8$ or $NR^8R^9$;
$R^5$ is hydrogen or $C_{1-6}$ alkyl optionally substituted by hydroxyl, $C_{1-6}$ alkoxy or $NR^8R^9$;
$R^6$ is hydrogen or $C_{1-6}$ alkyl;
$R^7$ is hydrogen or $C_{1-6}$ alkyl;
$R^8$ is hydrogen, $C_{1-6}$ alkyl or $C_{1-3}$ alkylphenyl wherein said phenyl groups are optionally substituted by one or more substituents selected from halogen, $C_{1-6}$ alkyl, $CF_3$, $OR^7$, $NR^8R^9$ or $OCF_3$;
$R^9$ is hydrogen, $C_{1-6}$ alkyl, $C_{1-6}$ alkenyl, phenyl or $C_{1-3}$ alkylphenyl wherein said phenyl groups are optionally substituted by one or more substituents selected from halogen, $C_{1-6}$ alkyl, $CF_3$, $OR^7$ or $OCF_3$;
or the groups $R^8$ and $R^9$ when they are attached to a nitrogen atom may together form a 5- or 6-membered ring which optionally contains one further heteroatom selected from $NR^7$, S and O and/or is optionally substituted by hydroxyl or $C_{1-6}$ alkoxy;
or the groups $R^8$ and $R^9$ when they are attached to a nitrogen atom may together form an azetidinyl ring optionally substituted by hydroxyl or $C_{1-6}$ alkoxy; and
$R^{10}$ is $C_{1-6}$ alkyl or a phenyl group optionally substituted by one or more substituents selected from halogen, $C_{1-6}$ alkyl, $CF_3$, $OCF_3$ or $OR^7$.

**5.** A compound as claimed in claim 1 or claim 2 selected from

8-Fluoro-4-[5-(3-morpholinophenoxy)pyrimidin-2-yl]-2,3-dihydro-1,4-benzoxazine;
6-Fluoro-4-[5-(3-morpholinophenoxy)pyrimidin-2-yl]-2,3-dihydro-1,4-benzoxazine;
6-Cyano-4-[5-(3-morpholinophenoxy)pyrimidin-2-yl]-2,3-dihydro-1,4-benzoxazine;
4-[5-(3-morpholinophenoxy)pyrimidin-2-yl]-2,3-dihydro-1,4-benzoxazine;
8-Fluoro-4-[5-(3-morpholinophenoxy)-2-pyridyl]-2,3-dihydro-1,4-benzoxazine;
9-Fluoro-5-[5-(3-morpholin-4-ylphenoxy)pyrimidin-2-yl]-2,3,4,5-tetrahydro-benzo-[b][1,4]oxazepine;
1-[5-(3-Morpholin-4-ylphenoxy)pyrimidin-2-yl]-2,3-dihydro-1H-pyrido-[2,3-b][1,4]-oxazine;
(S)-8-Fluoro-4-[5-(3-methylmorpholin-4-ylphenoxy)pyrimidin-2-yl]-2,3-dihydro-1,4-benzoxazine;
(R)-8-Fluoro-4-[5-(3-methylmorpholin-4-ylphenoxy)pyrimidin-2-yl]-2,3-dihydro-1,4-benzoxazine;
8-Fluoro-4-[5-(3-methoxyphenoxy)pyrimidin-2-yl]-2,3-dihydro-1,4-benzoxazine;
8-Fluoro-4-[5-(3-N,N-dimethylaminophenoxy)pyrimidin-2-yl]-2, 3-dihydro-1,4-benzoxazine;
4-((5-(3-bromophenoxy)pyrimidine-2yl)-8-fluoro-3,4-dihydro-2H-benzo[1,4]oxazine;
1-{3-[2-(8-Fluoro-2,3-dihydro-benzo[1,4]oxazin-4-yl)-pyrimidin-5-yloxy]-phenyl}azetidin-3-ol;
4-[5-(3-Morpholin-4-yl-phenoxy)pyridin-2-yl]-3,4-dihydro-2H-benzo[1,4]oxazine-6-carbonitrile; and
8-Fluoro-3-methyl-4-[5-(3-morpholin-4-yl-phenoxy)pyrimidin-2-yl]-3,4-dihydro-2H-benzo[1,4]oxazine

**6.** A pharmaceutical composition comprising a compound as claimed in any one of claims 1 to 5, optionally together with one or more pharmaceutically acceptable carriers or excipients.

7. A compound as claimed in any one of claims 1 to 5 or a pharmaceutical composition as claimed in claim 6 for use in the treatment of an amyloid-related disease.

8. A compound or pharmaceutical composition for use as claimed in claim 7 which is for use in the treatment of:

a) any form of Alzheimer's disease (AD or FAD);
b) any form of mild cognitive impairment (MCI) or senile dementia;
c) Down's syndrome;
d) cerebral amyloid angiopathy, inclusion body myositis, hereditary cerebral hemorrhage with amyloidosis (HCHWA, Dutch type), or age-related macular degeneration (ARMD);
e) fronto-temporal dementia;
f) any form of Parkinson's disease (PD) or dementia with Lewy bodies;
g) Huntington's disease (HD), dentatorubral pallidoluysian atrophy (DRPLA), spinocerebellar ataxia (SCA, types 1, 2, 3, 6 and 7), spinal and bulbar muscular atrophy (SBMA, Kennedy's disease), or any other polyglutamine disease;
h) Creutzfeldt-Jakob disease (CJD), bovine spongiform encephalopathy (BSE) in cows, scrapie in sheep, kuru, Gerstmann-Straussler-Scheinker disease (GSS), fatal familial insomnia, or any other transmissible encephalopathy that is associated with the aggregation of prion proteins;
i) amyotrophic lateral sclerosis (ALS) or any other form of motor neuron disease;
j) familial British dementia (FBD) or familial Danish dementia (FDD);
k) hereditary cerebral hemorrhage with amyloidosis (HCHWA, Icelandic type);
l) type II diabetes (adult onset diabetes, or non-insulin dependent diabetes mellitus, NIDDM);
m) dialysis-related amyloidosis (DRA) or prostatic amyloid;
n) primary systemic amyloidosis, systemic AL amyloidosis, or nodular AL amyloidosis;
o) myeloma associated amyloidosis;
p) systemic (reactive) AA amyloidosis, secondary systemic amyloidosis, chronic inflammatory disease, or familial Mediterranean fever;
q) senile systemic amyloidosis, familial amyloid polyneuropathy, or familial cardiac amyloid;
r) familial visceral amyloidosis, hereditary non-neuropathic systemic amyloidosis, or any other lysozyme-related amyloidosis;
s) Finnish hereditary systemic amyloidosis;
t) fibrinogen $\alpha$-chain amyloidosis;
u) insulin-related amyloidosis;
v) medullary carcinoma of the thyroid;
w) isolated atrial amyloidosis;
x) any form of cataract; or
y) any other amyloid-related disease that is associated with the misfolding or aggregation of a specific target amyloid-forming protein or peptide into toxic soluble oligomers, protofibrils, ion channels, insoluble amyloid fibres, plaques or inclusions.

**Patentansprüche**

1. Verbindung der Formel (1) oder pharmazeutisch akzeptables Salz davon:

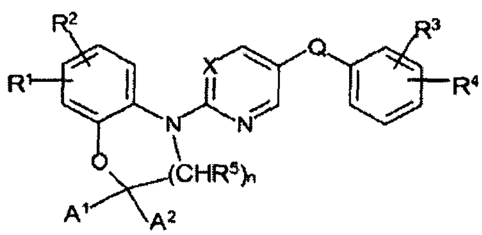

(I)

wobei

X N oder CH ist;

Q $NR^6$ oder O ist;
$A^1$ und $A^2$ unabhängig Wasserstoff oder $C_{1-6}$-Alkyl sind oder zusammen eine Carbonylgruppe bilden können;
$R^1$ und $R^2$ unabhängig Wasserstoff, Halogen, $CF_3$, CN, $OR^7$, $OR^8$, $NR^8R^9$, $NR^8COR^{10}$, $NR^8SO_2R^{10}$, $SO_2NR^8R^9$, $SO_2R^{10}$ oder $C_{1-6}$-Alkyl, wahlweise und unabhängig durch eines oder mehrere von Hydroxyl, $C_{1-6}$-Alkoxy, Halogen oder $NR^8R^9$ substituier, sind;
$R^3$ Wasserstoff, Halogen, $CF_3$ oder $OR^7$ ist;
$R^4$ Wasserstoff, Halogen, $CF_3$, $OR^8$, $NR^8R^9$, $NR^8COR^{10}$, $NR^BSO_2R^{10}$ oder $C4_{1-6}$-Alkyl, wahlweise durch Hydroxyl, $C_{1-6}$-Alkoxy oder $NR^8R^9$ substituiert, ist;
oder wenn $R^3$ und $R^4$ sich in der Ortho-Position befinden und zusammengenommen werden, $-O(CH_2)_mO$-bilden, wobei m 1 - 3 beträgt;
$R^5$ Wasserstoff oder $C_{1-6}$-Alkyl, wahlweise durch Hydroxyl, $C_{1-6}$-Alkoxy oder $NR^8R^9$ substituiert, ist;
$R^6$ Wasserstoff oder $C_{1-6}$-Alkyl ist;
$R^7$ Wasserstoff oder $C_{1-6}$-Alkyl, wahlweise durch $OR^8$ oder $NR^8R^9$ substituiert, ist;
$R^8$ Wasserstoff, $C_{1-6}$-Alkyl, wahlweise durch Hydroxyl oder $C_{1-6}$-Alkoxy oder $C_{1-3}$-Alkylphenyl substituiert, ist, wobei die Phenylgruppe wahlweise durch einen oder mehrere Substituenten substituiert ist ausgewählt unter Halogen, $C_{1-6}$-Alkyl, $CF_3$, $OR^7$, $NR^8R^9$ oder $OCF_3$;
$R^9$ Wasserstoff, $C_{1-6}$-Alkyl, $C_{1-6}$-Alkenyl, $C_{1-6}$-Alkynyl, Phenyl oder $C_{1-3}$-Alkylphenyl ist, wobei die Phenylgruppen wahlweise durch einen oder mehrere Substituenten substituiert sind ausgewählt unter Halogen, $C_{1-6}$-Alkyl, $CF_3$, $OR^7$, $NR^8R^9$ oder $OCF_3$;
oder die Gruppen $R^8$ und $R^9$, wenn sie an ein Stickstoffatom angelagert sind, zusammen einen 5- oder 6-gliedrigen Ring bilden können, der wahlweise ein weiteres Heteroatom enthält, ausgewählt unter $NR^7$, S und O, wobei der 5- oder 6-gliedrige Ring wahlweise durch Hydroxyl oder $C_{1-6}$-Alkoxy substituiert ist;
oder die Gruppen $R^8$ und $R^9$, wenn sie an ein Stickstoffatom angelagert sind, zusammen einen Azetidinylring bilden können, der wahlweise durch Hydroxyl oder $C_{1-6}$-Alkoxy substituiert ist; und
$R^{10}$ $C_{1-6}$-Alkyl oder eine Phenylgruppe ist, die wahlweise durch einen oder mehrere Substituenten substituiert ist ausgewählt unter Halogen, $C_{1-6}$-Alkyl, $CF_3$, $OCF_3$ oder $OR^7$; und
n 1 oder 2 beträgt.

**2.** Verbindung der Formel (1a) oder pharmazeutisch akzeptables Salz davon:

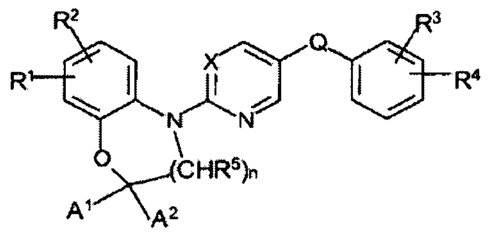

(Ia)

wobei

X N oder CH ist;
Q $NR^6$ oder O ist;
$A^1$ und $A^2$ unabhängig Wasserstoff oder $C_{1-6}$-Alkyl sind und zusammen eine Carbonylgruppe bilden können;
$R^1$ und $R^2$ unabhängig Wasserstoff, Halogen, $CF_3$, CN, $OR^7$, $OR^8$, $NR^8R^9$, $NR^8COR^{10}$, $NR^8SO_2R^{10}$, $SO_2NR^8R^9$, $SO_2R^{10}$ oder $C_{1-6}$-Alkyl und wahlweise und unabhängig durch eines oder mehrere von Hydroxyl, $C_{1-6}$-Alkoxy, Halogen oder $NR^8R^9$ substituiert sind;
$R^3$ Wasserstoff, Halogen, $CF_3$ oder $OR^7$ ist;
$R^4$ Wasserstoff, Halogen, $CF_3$, $OR^8$, $NR^8R^9$, $NR^8COR^{10}$, $NR^8SO_2R^{10}$ oder $C_{1-6}$-Alkyl, wahlweise durch Hydroxyl, $C_{1-6}$-Alkoxy oder $NR^8R^9$ substituiert, ist;
oder wenn $R^3$ und $R^4$ sich in der Ortho-Position befinden und zusammengenommen werden, $-O(CH_2)_mO$-bilden, wobei m 1 - 3 beträgt;
$R^5$ Wasserstoff oder $C_{1-6}$-Alkyl, wahlweise durch Hydroxyl, $C_{1-6}$-Alkoxy oder $NR^8R^9$ substituiert, ist;
$R^6$ Wasserstoff oder $C_{1-6}$-Alkyl ist;
$R^7$ Wasserstoff oder $C_{1-6}$-Alkyl, wahlweise durch $OR^8$ oder $NR^8R^9$ substituiert, ist;
$R^8$ Wasserstoff, $C_{1-6}$-Alkyl oder $C_{1-3}$-Alkylphenyl ist, wobei die Phenylgruppe wahlweise durch einen oder mehrere Substituenten substituiert ist ausgewählt unter Halogen, $C_{1-6}$-Alkyl, $CF_3$, $OR^7$, $NR^8R^9$ oder $OCF_3$;

$R^9$ Wasserstoff, $C_{1-6}$-Alkyl, $C_{1-6}$-Alkenyl, Phenyl oder $C_{1-3}$-Alkylphenyl ist, wobei die Phenylgruppen wahlweise durch einen oder mehrere Substituenten substituiert sind ausgewählt unter Halogen, $C_{1-6}$-Alkyl, $CF_3$, $OR^7$ oder $OCF_3$;
oder die Gruppen $R^8$ und $R^9$, wenn sie an ein Stickstoffatom angelagert sind, zusammen einen 5- oder 6-gliedrigen Ring bilden können, der wahlweise ein weiteres Heteroatom enthält, ausgewählt unter $NR^7$, S und O; und
$R^{10}$ $C_{1-6}$-Alkyl oder eine Phenylgruppe ist, die wahlweise durch einen oder mehrere Substituenten substituiert ist ausgewählt unter $C_{1-6}$-Alkyl, $CF_3$, $OCF_3$ oder $OR^7$; und n 1 oder 2 beträgt.

**3.** Verbindung nach Anspruch 1 oder Anspruch 2, wobei

X N oder CH ist;
Q $NR^6$ oder O ist;
$A^1$ und $A^2$ unabhängig Wasserstoff oder $C_{1-6}$-Alkyl sind und zusammen eine Carbonylgruppe bilden können;
m = 1 - 2 und n = 1 betragen;
$R^1$ und $R^2$ unabhängig Wasserstoff, Halogen, $CF_3$, CN, $OR^7$, $NR^8R^9$, $NR^8COR^{10}$, $NR^8SO_2R^{10}$ oder $C_{1-6}$-Alkyl, wahlweise durch Hydroxyl, $C_{1-6}$-Alkoxy oder $NR^8R^9$ substituiert, sind;
$R^3$ Wasserstoff, Halogen, $CF_3$ oder $OR^7$ ist;
$R^4$ Wasserstoff, Halogen, $CF_3$, $OR^8$, $NR^8R^9$, $NR^8COR^{10}$ oder $NR^8SO_2R^{10}$ ist;
$R^5$ Wasserstoff oder $C_{1-6}$-Alkyl, wahlweise durch Hydroxyl, $C_{1-6}$-Alkoxy oder $NR^8R^9$ substituiert, ist,
$R^6$ Wasserstoff oder $C_{1-6}$-Alkyl ist;
$R^7$ Wasserstoff oder $C_{1-6}$-Alkyl, wahlweise durch $OR^8$ oder $NR^8R^9$ substituiert, ist;
$R^8$ Wasserstoff, $C_{1-6}$-Alkyl oder $C_{1-3}$-Alkylphenyl ist, wobei die Phenylgruppe wahlweise durch einen oder mehrere Substituenten substituiert ist ausgewählt unter Halogen, $C_{1-6}$-Alkyl, $CF_3$, $OR^7$, $NR^8R^9$ oder $OCF_3$;
$R^9$ Wasserstoff, $C_{1-6}$-Alkyl, $C_{1-6}$-Alkenyl, Phenyl oder $C_{1-3}$-Alkylphenyl ist, wobei die Phenylgruppen wahlweise durch einen oder mehrere Substituenten substituiert sind ausgewählt unter Halogen, $C_{1-6}$-Alkyl, $CF_3$, $OR^7$ oder $OCF_3$;
oder die Gruppen $R^8$ und $R^9$, wenn sie an ein Stickstoffatom angelagert sind, zusammen einen 5- oder 6-gliedrigen Ring bilden können, der wahlweise ein weiteres Heteroatom enthält, ausgewählt unter $NR^7$, S und O und/oder wahlweise durch Hydroxyl oder $C_{1-6}$-Alkoxy substituiert ist;
oder die Gruppen $R^8$ und $R^9$, wenn sie an ein Stickstoffatom angelagert sind, zusammen einen Azetidinylring bilden können, der wahlweise durch Hydroxyl oder $C_{1-6}$-Alkoxy substituiert ist; und
$R^{10}$ $C_{1-6}$-Alkyl oder eine Phenylgruppe ist, die wahlweise durch einen oder mehrere Substituenten substituiert ist ausgewählt unter $C_{1-6}$-Alkyl, $CF_3$, $OCF_3$ oder $OR^7$.

**4.** Verbindung nach einem der Ansprüche 1 bis 3, wobei

X N oder CH ist;
Q $NR^6$ oder O ist;
$A^1$ und $A^2$ unabhängig Wasserstoff oder $C_{1-6}$-Alkyl sind oder zusammen eine Carbonylgruppe bilden können;
m = 1 - 2 und n = 1 betragen;
$R^1$ und $R^2$ unabhängig Wasserstoff, Halogen, $CF_3$, CN, $OR^7$ oder $NR^8R^9$ sind;
$R^3$ Wasserstoff, F oder $OR^7$ ist;
$R^4$ Wasserstoff, Halogen, $CF_3$, $OR^8$ oder $NR^8R^9$ ist;
$R^5$ Wasserstoff oder $C_{1-6}$-Alkyl, wahlweise durch Hydroxy, $C_{1-6}$-Alkoxy oder $NR^8R^9$ substituiert, ist;
$R^6$ Wasserstoff oder $C_{1-6}$-Alkyl ist;
$R^7$ Wasserstoff oder $C_{1-6}$-Alkyl ist,
$R^8$ Wasserstoff, $C_{1-6}$-Alkyl oder $C_{1-3}$-Alkylphenyl ist, wobei die Phenylgruppen wahlweise durch einen oder mehrere Substituenten substituiert sind ausgewählt unter Halogen, $C_{1-6}$-Alkyl, $CF_3$, $OR^7$, $NR^8R^9$ oder $OCF_3$;
$R^9$ Wasserstoff, $C_{1-6}$-Alkyl, $C_{1-6}$-Alkenyl, Phenyl oder $C_{1-3}$-Alkylphenyl ist, wobei die Phenylgruppen wahlweise durch einen oder mehrere Substituenten substituiert sind ausgewählt unter Halogen, $C_{1-6}$-Alkyl, $CF_3$, $OR^7$ oder $OCF_3$;
oder die Gruppen $R^8$ und $R^9$, wenn sie an ein Stickstoffatom angelagert sind, zusammen einen 5- oder 6-gliedrigen Ring bilden können, der wahlweise ein weiteres Heteroatom enthält, ausgewählt unter $NR^7$, S und O und/oder wahlweise durch Hydroxyl oder $C_{1-6}$-Alkoxy substituiert ist;
oder die Gruppen $R^8$ und $R^9$, wenn sie an ein Stickstoffatom angelagert sind, zusammen einen Azetidinylring bilden können, der wahlweise durch Hydroxyl oder $C_{1-6}$-Alkoxy substituiert ist; und
$R^{10}$ $C_{1-6}$-Alkyl oder eine Phenylgruppe ist, die wahlweise durch einen oder mehrere Substituenten substituiert

ist ausgewählt unter $C_{1-6}$-Alkyl, $CF_3$, $OCF_3$ oder $OR^7$.

**5.** Verbindung nach Anspruch 1 oder Anspruch 2, ausgewählt unter

8-Fluor-4-[5-(3-morpholinophenoxy)pyrimidin-2-yl]-2,3-dihydro-1,4-benzoxazin;
6-Fluor-4-[5-(3-morpholinophenoxy)pyrimidin-2-yl]-2,3-dihydro-1,4-benzoxazin;
6-Cyano-4-[5-(3-morpholinophenoxy)pyrimidin-2-yl]-2,3-dihydro-1,4-benzoxazin;
4-[5-(3-morpholinophenoxy)pyrimidin-2-yl]-2,3-dihydro-1,4-benzoxazin;
8-Fluor-4-[5-(3-morpholinophenoxy)-2-pyridyl]-2,3-dihydro-1,4-benzoxazin;
9-Fluor-5-[5-(3-morpholinophenoxy)pyrimidin-2-yl]-2,3,4,5-tetrahydrobenzo-[b][1,4]oxazepin;
1-[5-(3-Morpholin-4-ylphenoxy)pyrimidin-2-yl]-2,3-dihydro-1H-pyrido-[2,3-b][1,4]oxazin;
(S)-8-Fluor-4-[5-(3-methylmorpholin-4-ylphenoxy)pyrimidin-2-yl]-2,3-dihydro-1,4-benzoxazin; (R)-8-Fluor-4-[5-(3-methylmorpholin-4-ylphenoxy)pyrimidin-2-yl]-2,3-dihydro-1,4-benzoxazin; 8-Fluor-4-[5-(3-methoxyphenoxy)pyrimidin-2-yl]-2,3-dihydro-1,4-benzoxazin;
8-Fluor-4-[5-(3-N,N-dinethylaminophenoxy)pyrimidin-2-yl]-2,3-dihydro-1,4-benzoxazin;
4-[5-(3-Bromphenoxy)pyrimidin-2-yl]-8-fluor-3,4-dihydro-2H-benzo[1,4]oxazin;
1-{3-[2-(8-Fluor-2,3-dihydrobenzo[1,4]oxazin-4-yl)-pyrimidin-5-yloxy[-phenyl}azetidin-3-ol;
4-[5[3-Morpholin-4-yl-phenoxy)pyridin-2-yl]-3,4-dihydro-2H-benzo[1,4]oxazin-6-carbonitril; und
8-Fluor-3-methyl-4-[5-(3-morpholin-4-yl-phenoxy)pyrimidin-2-yl]-4,3-dihydro-2H-benzo[1,4]-oxazin.

**6.** Pharmazeutische Zusammensetzung umfassend eine Verbindung nach einem der Ansprüche 1 bis 5, wahlweise zusammen mit einem oder mehreren pharmazeutisch akzeptablen Trägern oder Hilfsstoffen.

**7.** Verbindung nach einem der Ansprüche 1 bis 5 oder pharmazeutisch akzeptable Zusammensetzung nach Anspruch 6 zur Verwendung bei der Behandlung einer mit amyloidverbundenen Krankheit.

**8.** Verbindung oder pharmazeutische Zusammensetzung zur Verwendung nach Anspruch 7, die zur Verwendung bei der Behandlung dient von:

a) irgendeiner Form von Alzheimerkrankheit (AD oder FAD);
b) irgendeiner Form von leichter kognitiver Beeinträchtigung (MCI) oder Altersdemenz;
c) Down-Syndrom;
d) zerebraler Amyloidangiopatie, Einschlusskörpermyositis, hereditärer Hirnblutung mit Amyloidose (HCHWA, holländischem Typ) oder altersbedingter Makuladegeneration (ARMD):
e) frontotemporaler Demenz;
f) irgendeiner Form von Parkinson-Krankheit (PD) oder Demenz mit Lewy-Körpern;
g) Huntington-Krankheit (HD), dentatorubropallidoluysischer Atrophie (DRPLA), spinozerebellärer Ataxie (SCA, Typen 1, 2, 3, 6 und 7), spinobilbärer Muskelatrophie (SBMA, Kennedy-Krankheit) oder irgendeiner anderen Polyglutaminkrankheit;
h) Creutzfeldt-Jakob-Krankheit (CJD), Rinderwahnsinn (BSE) bei Kühen, Traberkrankheit bei Schafen, Kuru, Gerstmann-Sträussler-Scheinker-Syndrom (GSS), tödlicher familiärer Schlaflosigkeit oder irgendwelcher übertragbarer Enzephalopathie die mit der Aggregation von Prion-Proteinen verbunden ist;
i) amyotrophen Lateralsklerose (ALS) oder irgendeiner anderen Form von Motorneuronerkrankung;
j) familiärer Britischer Demenz (FBD) oder familiärer Dänischer Demenz (FDD);
k) erblicher Hirnblutung mit Amyloidose (HCHWA, Isländischem Typ);
l) Typ-2-Diabetes (Altersdiabetes oder nicht insulinabhängiger Diabetes mellitus, NIDDM);
m) mit Dialyse verbundener Amyloidose (DRA) oder Prostataamyloidose;
n) primärer, systemischer Amyloidose, systemischer AL-Amyloidose oder nodulärer Amyloidose;
o) mit Amyloidose verbundenem Myelom;
p) systemischer (reaktiver) AA-Amyloidose, sekundärer systemischer Amyloidose, chronischer Entzündungskrankheit oder familiärem Mittelmeerfieber;
q) seniler systemischer Amyloidose, familiärer Amyloidpolyneuropathie oder familiärem Kardioamyloid;
r) familiärer viszeraler Amyloidose, erblicher nichtneuropathischer systemischer Amyloidose oder irgendeiner anderen lysozymverbundenen Amyloidose;
s) Finnischer erblicher systemischer Amyloidose;
t) Fibrinogen-$\alpha$-Ketten-Amyloidose;
u) mit Insulin verbundener Amyloidose;
v) medullärem Karzinom der Schilddrüse;

w) isolierter atrialer Amyloidose;

x) irgendeiner Form von grauem Star; oder

y) irgendeiner anderen amyloidbedingter Krankheit, die mit dem Fehlfalten oder der Aggregation eines spezifischen amyloidbildenden Targetproteins oder -peptids in toxische lösliche Oligomere, Protofibrillen, Ionenkanäle, unlösliche Amyloidfasern, Plaques oder Einschlüsse verbunden ist.

## Revendications

**1.** Composé de formule (I) ou sel pharmaceutiquement acceptable de celui-ci :

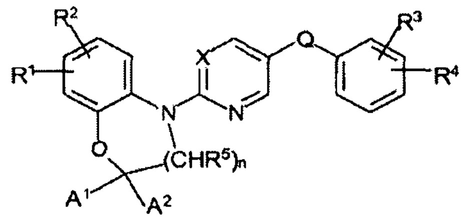

(I)

dans laquelle

X est N ou CH ;

Q est $NR^6$ ou O ;

$A^1$ et $A^2$ sont indépendamment un atome d'hydrogène ou un groupe alkyle en $C_1$ à $C_6$ ou peuvent former conjointement un groupe carbonyle ;

$R^1$ et $R^2$ sont indépendamment un atome d'hydrogène, d'halogène, $CF_3$, CN, $OR^7$, $OR^8$, $NR^8R^9$, $NR^8COR^{10}$, $NR^8SO_2R^{10}$, $SO_2NR^8R^9$, $SO_2R^{10}$ ou alkyle en $C_1$ à $C_6$ éventuellement et indépendamment substitués par un ou plusieurs groupes hydroxyle, alcoxy en $C_1$ à $C_6$, halogéno ou $NR^8R^9$;

$R^3$ est un atome d'hydrogène, d'halogène, $CF_3$ ou $OR^7$;

$R^9$ est un atome d'hydrogène, d'halogène, $CF_3$, $OR^8$, $NR^8R^9$, $NR^8COR^{10}$, $NR^8SO_2R^{10}$ ou alkyle en $C_1$ à $C_6$ éventuellement substitué par un groupe hydroxyle, alcoxy en $C_1$ à $C_6$ ou $NR^8R^9$;

ou dans lequel $R^3$ et $R^4$ sont en position ortho et pris conjointement, forment $-O(CH_2)_mO-$, où m vaut de 1 à 3 ;

$R^5$ est un atome d'hydrogène ou un groupe alkyle en $C_1$ à $C_6$ éventuellement substitué par un groupe hydroxyle, alcoxy en $C_1$ à $C_6$ ou $NR^8R^9$ ;

$R^6$ est un atome d'hydrogène ou un groupe alkyle en $C_1$ à $C_6$ ;

$R^7$ est un atome d'hydrogène ou un groupe alkyle en $C_1$ à $C_6$ éventuellement substitué par $OR^8$ ou $NR^8R^9$ ;

$R^8$ est un atome d'hydrogène, un groupe alkyle en $C_1$ à $C_6$, éventuellement substitué par un groupe hydroxyle ou alcoxy en $C_1$ à $C_6$ ou alkylphényle en $C_1$ à $C_3$, dans lequel ledit groupe phényle est éventuellement substitué par un ou plusieurs substituants choisis parmi un atome d'halogène, un groupe alkyle en $C_1$ à $C_6$, $CF_3$, $OR^7$, $NR^8R^9$ ou $OCF_3$ ;

$R^9$ est un atome d'hydrogène, un groupe alkyle en $C_1$ à $C_6$, alcényle en $C_1$ à $C_6$, alcinyle en $C_1$ à $C_6$, phényle ou alkylphényle en $C_1$ à $C_3$, dans lequel lesdits groupes phényle sont éventuellement substitués par un ou plusieurs substituants choisis parmi un atome d'halogène, un groupe alkyle en $C_1$ à $C_6$, $CF_3$, $OR^7$ ou $OCF_3$ ;

ou les groupes $R^8$ et $R^9$, lorsqu'ils sont fixés à un atome d'azote, peuvent former conjointement un cycle de 5 ou 6 chaînons qui contient éventuellement un autre hétéroatome choisi parmi $NR^7$, S et O, ledit cycle de 5 ou 6 chaînons étant éventuellement substitué par un groupe hydroxyle ou alcoxy en $C_1$ à $C_6$ ;

ou les groupes $R^8$ et $R^9$, lorsqu'ils sont fixés à un atome d'azote, peuvent former conjointement un cycle azétidinyle éventuellement substitué par un groupe hydroxyle ou alcoxy en $C_1$ à $C_6$ ; et

$R^{10}$ est un groupe alkyle en $C_1$ à $C_6$ ou phényle éventuellement substitué par un ou plusieurs substituants choisis parmi un atome d'halogène, un groupe alkyle en $C_1$ à $C_6$, $CF_3$, $OCF_3$ ou $OR^7$ ; et

n vaut 1 ou 2.

**2.** Composé de formule (Ia) ou sel pharmaceutiquement acceptable de celui-ci :

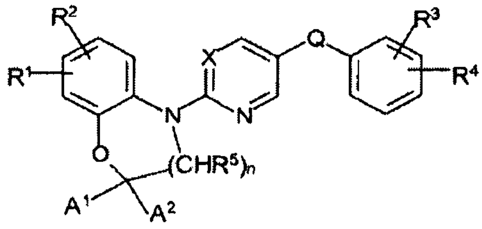

(Ia)

dans laquelle

X est N ou CH ;
Q est $NR^6$ ou O ;
$A^1$ et $A^2$ sont indépendamment un atome d'hydrogène ou un groupe alkyle en $C_1$ à $C_6$ ou peuvent former conjointement un groupe carbonyle ;
$R^1$ et $R^2$ sont indépendamment un atome d'hydrogène, d'halogène, $CF_3$, CN, $OR^7$, $OR^8$, $NR^8R^9$, $NR^8COR^{10}$, $NR^8SO_2R^{10}$, $SO_2NR^8R^9$, $SO_2R^{10}$ ou alkyle en $C_1$ à $C_6$ éventuellement et indépendamment substitués par un ou plusieurs groupes hydroxyle, alcoxy en $C_1$ à $C_6$, halogéno ou $NR^8R^9$ ;
$R^3$ est un atome d'hydrogène, d'halogène, $CF_3$ ou $OR^7$;
$R^9$ est un atome d'hydrogène, d'halogène, $CF_3$, $OR^8$, $NR^8R^9$, $NR^8COR^{10}$, $NR^8SO^2R^{10}$ ou alkyle en $C_1$ à $C_6$ éventuellement substitué par un groupe hydroxyle, alcoxy en $C_1$ à $C_6$ ou $NR^8R^9$ ;
ou dans lequel $R^3$ et $R^4$ sont en position ortho et pris conjointement, forment $-O(CH_2)_mO-$, où m vaut de 1 à 3 ;
$R^5$ est un atome d'hydrogène ou un groupe alkyle en $C_1$ à $C_6$ éventuellement substitué par un groupe hydroxyle, alcoxy en $C_1$ à $C_6$ ou $NR^8R^9$ ;
$R^6$ est un atome d'hydrogène ou un groupe alkyle en $C_1$ à $C_6$ ;
$R^7$ est un atome d'hydrogène ou un groupe alkyle en $C_1$ à $C_6$ éventuellement substitué par $OR^8$ ou $NR^8R^9$ ;
$R^8$ est un atome d'hydrogène, un groupe alkyle en $C_1$ à $C_6$ ou alkylphényle en $C_1$ à $C_3$, dans lequel ledit groupe phényle est éventuellement substitué par un ou plusieurs substituants choisis parmi un atome d'halogène, un groupe alkyle en $C_1$ à $C_6$, $CF_3$, $OR^7$, $NR^8R^9$ ou $OCF_3$ ;
$R^9$ est un atome d'hydrogène, un groupe alkyle en $C_1$ à $C_6$, alcényle en $C_1$ à $C_6$, phényle ou alkylphényle en $C_1$ à $C_3$, dans lequel lesdits groupes phényle sont éventuellement substitués par un ou plusieurs substituants choisis parmi un atome d'halogène, un groupe alkyle en $C_1$ à $C_6$, $CF_3$, $OR^7$ ou $OCF_3$ ;
ou les groupes $R^8$ et $R^9$, lorsqu'ils sont fixés à un atome d'azote, peuvent former conjointement un cycle de 5 ou 6 chaînons qui contient éventuellement un autre hétéroatome choisi parmi $NR^7$, S et O, et
$R^{10}$ est un groupe alkyle en $C_1$ à $C_6$ ou phényle éventuellement substitué par un ou plusieurs substituants choisis parmi un atome d'halogène, un groupe alkyle en $C_1$ à $C_6$, $CF_3$, $OCF_3$ ou $OR^7$ ; et
n vaut 1 ou 2.

**3.** Composé selon la revendication 1 ou la revendication 2, dans lequel

X est N ou CH ;
Q est $NR^6$ ou O ;
$A^1$ et $A^2$ sont indépendamment un atome d'hydrogène ou un groupe alkyle en $C_1$ à $C_6$ ou peuvent former conjointement un groupe carbonyle ;
m = 1 à 2 et n = 1 ;
$R^1$ et $R^2$ sont indépendamment un atome d'hydrogène, d'halogène, $CF_3$, CN, $OR^7$, $NR^8R^9$, $NR^8COR^{10}$, $NR^8SO_2R^{10}$ ou un groupe alkyle en $C_1$ à $C_6$ éventuellement substitué par un groupe hydroxyle, alcoxy en $C_1$ à $C_6$ ou $NR^8R^9$ ;
$R^3$ est un atome d'hydrogène, d'halogène, $CF_3$ ou $OR^7$ ;
$R^4$ est un atome d'hydrogène, d'halogène, $CF_3$, $OR^8$, $NR^8R^9$, $NR^8COR^{10}$ ou $NR^8SO_2R^{10}$ ;
$R^5$ est un atome d'hydrogène ou un groupe alkyle en $C_1$ à $C_6$ éventuellement substitué par un groupe hydroxyle, alcoxy en $C_1$ à $C_6$ ou $NR^8R^9$;
$R^6$ est un atome d'hydrogène ou un groupe alkyle en $C_1$ à $C_6$ ;
$R^7$ est un atome d'hydrogène ou un groupe alkyle en $C_1$ à $C_6$ éventuellement substitué par $OR^8$ ou $NR^8R^9$ ;
$R^8$ est un atome d'hydrogène, un groupe alkyle en $C_1$ à $C_6$ ou alkylphényle en $C_1$ à $C_3$, dans lequel ledit groupe phényle est éventuellement substitué par un ou plusieurs substituants choisis parmi un atome d'halogène, un groupe alkyle en $C_1$ à $C_6$, $CF_3$, $OR^7$, $NR^8R^9$ ou $OCF_3$ ;

$R^9$ est un atome d'hydrogène, un groupe alkyle en $C_1$ à $C_6$, alcényle en $C_1$ à $C_6$, phényle ou alkylphényle en $C_1$ à $C_3$, dans lequel lesdits groupes phényle sont éventuellement substitués par un ou plusieurs substituants choisis parmi un atome d'halogène, un groupe alkyle en $C_1$ à $C_6$, $CF_3$, $OR^7$ ou $OCF_3$ ;
ou les groupes $R^8$ et $R^9$, lorsqu'ils sont fixés à un atome d'azote, peuvent former conjointement un cycle de 5 ou 6 chaînons qui contient éventuellement un autre hétéroatome choisi parmi $NR^7$, S et O et/ou est éventuellement substitué par un groupe hydroxyle ou alcoxy en $C_1$ à $C_6$ ;
ou les groupes $R^8$ et $R^9$, lorsqu'ils sont fixés à un atome d'azote, peuvent former conjointement un cycle azétidinyle éventuellement substitué par un groupe hydroxyle ou alcoxy en $C_1$ à $C_6$ ; et
$R^{10}$ est un groupe alkyle en $C_1$ à $C_6$ ou phényle éventuellement substitué par un ou plusieurs substituants choisis parmi un atome d'halogène, un groupe alkyle en $C_1$ à $C_6$, $CF_3$, $OCF_3$ ou $OR^7$.

**4.** Composé selon l'une quelconque des revendications 1 à 3, dans lequel

X est N ou CH ;
Q est $NR^6$ ou O ;
$A^1$ et $A^2$ sont indépendamment un atome d'hydrogène ou un groupe alkyle en $C_1$ à $C_6$ ou peuvent former conjointement un groupe carbonyle ;
m = 1 à 2 et n = 1 ;
$R^1$ et $R^2$ sont indépendamment un atome d'hydrogène, d'halogène, $CF_3$, CN, $OR^7$ ou $NR^8R^9$;
$R^3$ est un atome d'hydrogène, F ou $OR^7$ ;
$R^4$ est un atome d'hydrogène, d'halogène, $CF_3$, $OR^8$ ou $NR^8R^9$ ;
$R^5$ est un atome d'hydrogène ou un groupe alkyle en $C_1$ à $C_6$ éventuellement substitué par un groupe hydroxyle, alcoxy en $C_1$ à $C_6$ ou $NR^8R^9$ ;
$R^6$ est un atome d'hydrogène ou un groupe alkyle en $C_1$ à $C_6$ ;
$R^7$ est un atome d'hydrogène ou un groupe alkyle en $C_1$ à $C_6$ ;
$R^8$ est un atome d'hydrogène, un groupe alkyle en $C_1$ à $C_6$ ou alkylphényle en $C_1$ à $C_3$, dans lequel lesdits groupes phényle sont éventuellement substitués par un ou plusieurs substituants choisis parmi un atome d'halogène, un groupe alkyle en $C_1$ à $C_6$, $CF_3$, $OR^7$, $NR^8R^9$ ou $OCF_3$ ;
$R^9$ est un atome d'hydrogène, un groupe alkyle en $C_1$ à $C_6$, alcényle en $C_1$ à $C_6$, phényle ou alkylphényle en $C_1$ à $C_3$, dans lequel lesdits groupes phényle sont éventuellement substitués par un ou plusieurs substituants choisis parmi un atome d'halogène, un groupe alkyle en $C_1$ à $C_6$, $CF_3$, $OR^7$ ou $OCF_3$ ;
ou les groupes $R^8$ et $R^9$, lorsqu'ils sont fixés à un atome d'azote, peuvent former conjointement un cycle de 5 ou 6 chaînons qui contient éventuellement un autre hétéroatome choisi parmi $NR^7$, S et O et/ou est éventuellement substitué par un groupe hydroxyle ou alcoxy en $C_1$ à $C_6$ ;
ou les groupes $R^8$ et $R^9$, lorsqu'ils sont fixés à un atome d'azote, peuvent former conjointement un cycle azétidinyle éventuellement substitué par un groupe hydroxyle ou alcoxy en $C_1$ à $C_6$ ; et
$R^{10}$ est un groupe alkyle en $C_1$ à $C_6$ ou phényle, éventuellement substitué par un ou plusieurs substituants choisis parmi un atome d'halogène, un groupe alkyle en $C_1$ à $C_6$, $CF_3$, $OCF_3$ ou $OR^7$.

**5.** Composé selon la revendication 1 ou la revendication 2, choisi parmi

la 8-fluoro-4-[5-(3-morpholinophénoxy) pyrimidin-2-yl]-2,3-dihydro-1,4-benzoxazine ;
la 6-fluoro-4-[5-(3-morpholinophénoxy) pyrimidin-2-yl]-2,3-dihydro-1,4-benzoxazine ;
la 6-cyano-4-[5-(3-morpholinophénoxy) pyrimidin-2-yl]-2,3-dihydro-1,4-benzoxazine ;
la 4-[5-(3-morpholinophénoxy)pyrimidin-2-yl]-2,3-dihydro-1,4-benzoxazine ;
la 8-fluoro-4-[5-(3-morpholinophénoxy)-2-pyridyl]-2,3-dihydro-1,4-benzoxazine ;
la 9-fluoro-5-[5-(3-morpholin-4-ylphénoxy) pyrimidin-2-yl]-2,3,4,5-tétrahydro-benzo[b][1,4] oxazépine ;
la 1-[5-(3-morpholin-4-ylphénoxy)pyrimidin-2-yl]-2,3-dihydro-1H-pyrido-[2,3-b][1,4]-oxazine;
la (S)-8-fluoro-4-[5-(3-méthylmorpholin-4-ylphénoxy)pyrimidin-2-yl]-2,3-dihydro-1,4-benzoxazine ;
la (R)-8-fluoro-4-[5-(3-méthylmorpholin-4-ylphénoxy)pyrimidin-2-yl]-2,3-dihydro-1,4-benzoxazine ;
la 8-fluoro-4-[5-(3-méthoxyphénoxy)pyrimidin-2-yl]-2,3-dihydro-1,4-benzoxazine ;
la 8-fluoro-4-[5-(3-N,N-diméthylaminophénoxy) pyrimidin-2-yl]-2,3-dihydro-1,4-benzoxazine ;
la 4-(5-(3-bromophénoxy)pyrimidin-2-yl]-8-fluoro-3,4-dihydro-2H-benzo[1,4]-benzoxazine ;
le 1-{3-[2-(8-fluoro-2,3-dihydro-benzo[1,4] oxazin-4-yl)-pyrimidin-5-yloxy]-phényl}-azétidin-ol ;
le 4-[5-(3-morpholin-4-yl-phénoxy)pyridin-2-yl]-3,4-dihydro-2H-benzo[1,4]oxazin-6-carbonitrile ; et
la 8-fluoro-3-méthyl-4-[5-(3-morpholin-4-yl-phénoxy)pyrimidin-2-yl]-3,4-dihydro-2H-benzo[1,4] oxazine.

**6.** Composition pharmaceutique comprenant un composé selon l'une quelconque des revendications 1 à 5, éventuellement conjointement à un ou plusieurs véhicules ou excipients pharmaceutiquement acceptables.

**7.** Composé selon l'une quelconque des revendications 1 à 5, ou composition pharmaceutique selon la revendication 6 pour son utilisation dans le traitement d'une maladie associée à l'amyloïde.

**8.** Composé ou composition pharmaceutique pour son utilisation selon la revendication 7, qui est destinée à l'utilisation dans le traitement de :

a) toute forme de la maladie d'Alzheimer (MA ou MAF) ;
b) toute forme de déficit cognitif léger (DCL) ou de démence sénile ;
c) du syndrome de Down ;
d) l'angiopathie amyloïde cérébrale, la myosite à corps d'inclusion, l'hémorragie cérébrale héréditaire avec amylose (HCHWA, type néerlandais) ou dégénérescence maculaire liée à l'âge (DMLA) ;
e) la démence fronto-temporale ;
f) toute forme de maladie de Parkinson (MP) ou de démence avec corps de Lewy ;
g) la maladie de Huntington (MH), l'atrophie dentato-rubro-pallido-luysienne (ADRPL), l'ataxie spino-cérébelleuse (ASC, types 1, 2, 3, 6 et 7), l'amyotrophie spinale et bulbaire (ASB, maladie de Kennedy) ou toute autre maladie liée à la polyglutamine ;
h) la maladie de Creuzfeld-Jacob (MKJ), l'encéphalopathie spongiforme bovine (ESB) chez les bovins, la tremblante du mouton, le kuru, la maladie de Gerstmann-Straussler-Scheinker (GSS), l'insomnie familiale fatale ou toute autre encéphalopathie transmissible qui est associée à l'agrégation des protéines de prions ;
i) la sclérose latérale amyotrophique (SLA) ou toute autre forme de maladie neuro-motrice ;
j) la démence britannique familiale (DBF) ou la démence danoise familiale (DDF) ;
k) l'hémorragie cérébrale héréditaire avec amylose (HCHWA, de type islandais) ;
l) le diabète de type II (diabète de l'adulte ou diabète sucré non insulino-dépendant (DSNID)
m) l'amyloïdose associée à la dialyse (AAD) ou l'amyloïdose prostatique ;
n) l'amyloïdose systémique primaire, l'amyloïdose systémique AL ou l'amyloïdose nodulaire AL ;
o) l'amyloïdose associée au myélome ;
p) l'amyloïdose AA systémique (réactive), l'amyloïdose systémique secondaire, la maladie chronique inflammatoire ou la fièvre méditerranéenne familiale ;
q) l'amyloïdose systémique sénile, la polyneuropathie amyloïde familiale ou l'amylose cardiaque familiale ;
r) l'amyloïdose viscréale familiale, l'amyloïdose systémique non neuropathique héréditaire ou toute autre amyloïdose associée au lysozyme ;
s) l'amyloïdose systémique héréditaire finlandaise ;
t) l'amyloïdose à chaîne $\alpha$ du fibrinogène ;
u) l'amyloïdose associée à l'insuline ;
v) le carcinome médullaire de la thyroïde ;
w) l'amyloïdose auriculaire isolée ;
x) toute forme de cataracte ; ou
y) toute autre maladie associée à l'amyloïde qui est associée au défaut de repliement ou à l'agrégation d'une protéine ou d'un peptide formant l'amyloïde cible dans des oligomères solubles toxiques, des protofibrilles, des canaux ioniques, des fibres, plaques ou inclusions d'amyloïde insolubles.

Figure 1

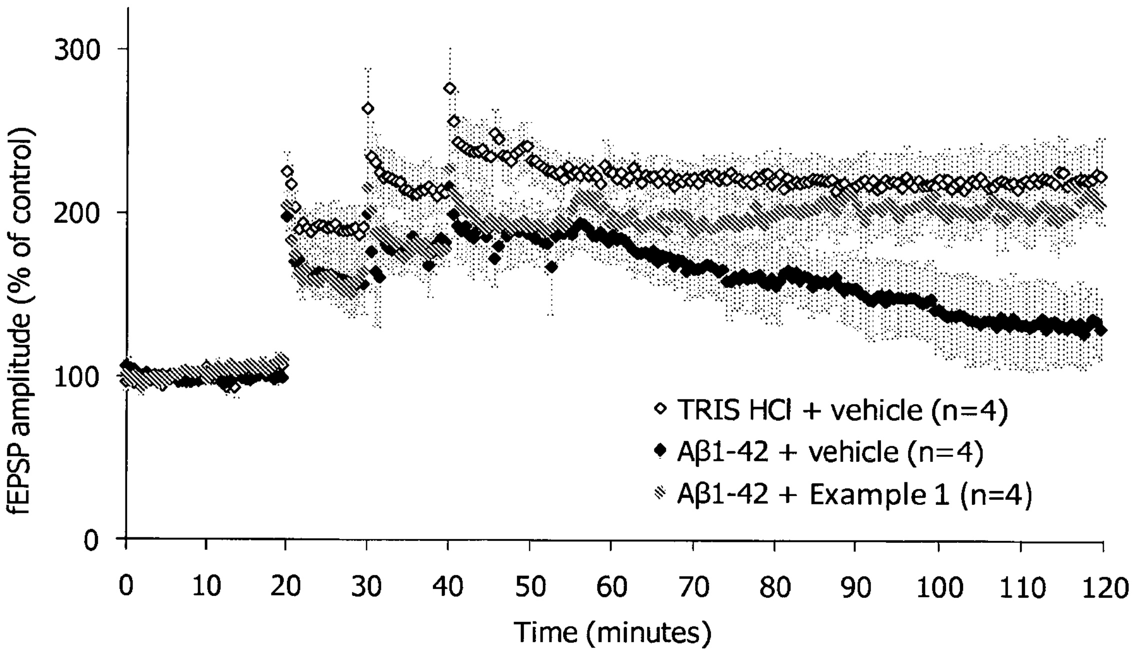
300
200
100
0
fEPSP amplitude (% of control)
0
10
20
30
40
50
60
70
80
90
100
110
120
Time (minutes)
TRIS HCl + vehicle (n=4)
Aβ1-42 + vehicle (n=4)
Aβ1-42 + Example 1 (n=4)

Figure 2

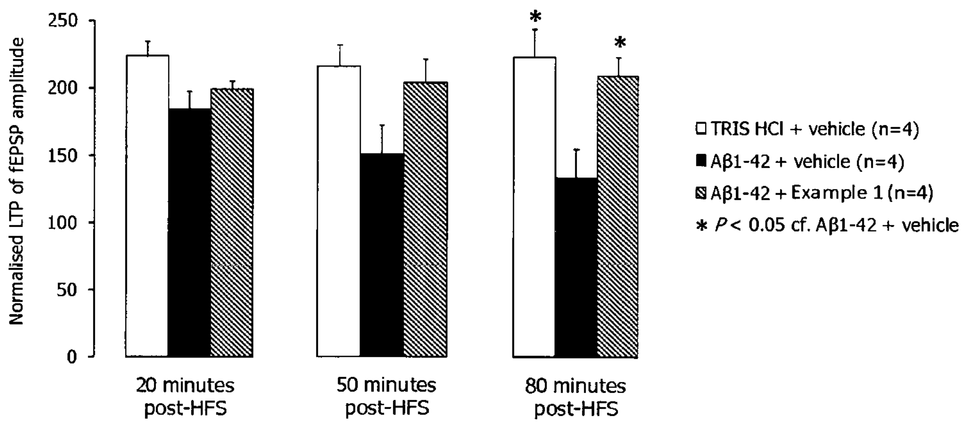
Normalised LTP of fEPSP amplitude
250
200
150
100
50
0
20 minutes post-HFS
50 minutes post-HFS
80 minutes post-HFS
*
*
TRIS HCl + vehicle (n=4)
Aβ1-42 + vehicle (n=4)
Aβ1-42 + Example 1 (n=4)
* P< 0.05 cf. Aβ1-42 + vehicle

Figure 3

| Condition | TRIS HCl + vehicle | Aβ1-42 + vehicle | Aβ1-42 + Example 1 |
|---|---|---|---|
| Mean normalised fEPSP amplitude 20 minutes post-HFS | 223.6 | 184.0 | 199.2 |
| SEM | 10.9 | 13.5 | 5.8 |
| Unpaired *t*-test *P* value cf. Aβ1-42 | 0.063 | - | 0.341 |
| Mean normalised fEPSP amplitude 50 minutes post-HFS | 217.8 | 152.0 | 205.0 |
| SEM | 16.1 | 21.6 | 18.0 |
| Unpaired *t*-test *P* value cf. Aβ1-42 | 0.050 | - | 0.108 |
| Mean normalised fEPSP amplitude 80 minutes post-HFS | 221.3 | 132.0 | 207.6 |
| SEM | 20.9 | 21.6 | 13.9 |
| Unpaired *t*-test *P* value cf. Aβ1-42 | 0.025 | - | 0.026 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009044160 A
- WO 2007079173 A
- WO 2005080361 A
- EP 0055583 A
- WO 2009121914 A

### Non-patent literature cited in the description


- **BUXBAUM, J. N.** The systemic amyloidoses. *Curr Opin Rheumatol,* 2004, vol. 16, 67-75
- **CAUGHEY, B. ; P. T. LANSBURY.** Protofibrils, pores, fibrils, and neurodegeneration: separating the responsible protein aggregates from the innocent bystanders. *Annu Rev Neurosci,* 2003, vol. 26, 267-298
- **CLEARY, J. P. ; D. M. WALSH ; J. J. HOFMEISTER ; G. M. SHANKAR ; M. A. KUSKOWSKI ; D. J. SELKOE ; K. H. ASHE.** Natural oligomers of the amyloid-β protein specifically disrupt cognitive function. *Nat Neurosci,* 2005, vol. 8, 79-84
- **DEV, K. K. ; K. HOFELE ; S. BARBIERI ; V. L. BUCHMAN ; H. VAN DER PUTTEN.** Part II: alpha-synuclein and its molecular pathophysiological role in neurodegenerative disease. *Neuropharmacology,* 2003, vol. 45, 14-44
- **ESTRADA, L. D. ; C. SOTO.** Disrupting β-amyloid aggregation for Alzheimer's disease treatment. *Curr Topics Med Chem,* 2007, vol. 7, 115-126
- **FORMAN, M. S. ; J. Q. TROJANOWSKI ; V. M. LEE.** Neurodegenerative diseases: a decade of discoveries paves the way for therapeutic breakthroughs. *Nat Med,* 2004, vol. 10, 1055-1063
- **GEJYO, F. ; T. YAMADA ; S. ODANI ; Y. NAKAGAWA ; M. ARAKAWA ; T. KUNITOMO ; H. KATAOKA ; M. SUZUKI ; Y. HIRASAWA ; T. SHIRAHAMA et al.** A new form of amyloid protein associated with chronic hemodialysis was identified as beta 2-microglobulin. *Biochem Biophys Res Commun,* 1985, vol. 129, 701-706
- **GLABE, C. G.** Conformation-dependent antibodies target diseases of protein misfolding. *Trends Biochem Sci,* 2004, vol. 29, 542-547
- **GLENNER, G. G.** Amyloid deposits and amyloidosis: the beta-fibrilloses. *N Engl J Med,* 1980, vol. 302, 1283-1292
- **GLENNER, G. G.** Amyloid deposits and amyloidosis: the beta-fibrilloses. *N Engl J Med,* 1980, vol. 302, 1333-1343
- **HAATAJA, L. ; T. GURLO ; C. J. HUANG ; P. C. BUTLER.** Islet amyloid in type 2 diabetes, and the toxic oligomer hypothesis. *Endocrine Rev,* 2008, vol. 29, 303-316
- **JAIKARAN, E. T. ; A. CLARK.** Islet amyloid and type 2 diabetes: from molecular misfolding to islet pathophysiology. *Biochim Biophys Acta,* 2001, vol. 1537, 179-203
- **KAGAN, B. L. ; Y. HIRAKURA ; R. AZIMOV ; R. AZIMOVA ; M. C. LIN.** The channel hypothesis of Alzheimer's disease: current status. *Peptides,* 2002, vol. 23, 1311-1315
- **KAYED, R. ; E. HEAD ; J. L. THOMPSON ; T. M. MCINTIRE ; S. C. MILTON ; C. W. COTMAN ; C. G. GLABE.** Common structure of soluble amyloid oligomers implies common mechanism of pathogenesis. *Science,* 2003, vol. 300, 486-489
- **LEVINE, H., III.** The amyloid hypothesis and the clearance and degradation of Alzheimer's beta-peptide. *J Alzheimers Dis,* 2004, vol. 6, 303-314
- Soluble amyloid beta peptide concentration as a predictor of synaptic change in Alzheimer's disease. *Am J Pathol,* 1999, vol. 155, 853-862
- Soluble pool of Abeta amyloid as a determinant of severity of neurodegeneration in Alzheimer's disease. *Ann Neurol,* 1999, vol. 46, 860-866
- **MASINO, L.** Polyglutamine and neurodegeneration: structural aspects. *Protein Pept Lett,* 2004, vol. 11, 239-248
- **MELNIKOVA, I.** Therapies for Alzheimer's disease. *Nat Rev Drug Disc,* 2007, vol. 6, 341-342
- **ROSS, C. A. ; M. A. POIRIER.** Protein aggregation and neurodegenerative disease. *Nat Med,* 2004, vol. 10, 10-7
- **SELKOE, D. J.** Folding proteins in fatal ways. *Nature,* 2003, vol. 426, 900-904
- **SHAH, R. S. ; H. G. LEE ; Z. XIONGWEI ; G. PERRY ; M. A. SMITH ; R. J. CASTELLANI.** Current approaches in the treatment of Alzheimer's disease. *Biomed Pharmacother,* 2008, vol. 62, 199-207

- **SHEARMAN, M. S.** Toxicity of protein aggregates in PC12 cells: 3-(4,5-dimethylthiazol-2-yl)-2,5-diphenyltetrazolium bromide assay. *Methods Enzymol,* 1999, vol. 309, 716-723
- **SOTO, C. ; J. CASTILLA.** The controversial protein-only hypothesis of prion propagation. *Nat Med,* 2004, vol. 10, 63-7
- **STEFANI, M.** Protein misfolding and aggregation: new examples in medicine and biology of the dark side of the protein world. *Biochim Biophys Acta,* 2004, vol. 1739, 5-25
- **TAYLOR, J. P. ; J. HARDY ; K. H. FISCHBECK.** Toxic proteins in neurodegenerative disease. *Science,* 2002, vol. 296, 1991-1995
- **WALSH, D. M. ; I. KLYUBIN ; J. V. FADEEVA ; W. K. CULLEN ; R. ANWYL ; M. S. WOLFE ; M. J. ROWAN ; D. J. SELKOE.** Naturally secreted oligomers of amyloid beta protein potently inhibit hippocampal long-term potentiation in vivo. *Nature,* 2002, vol. 416, 535-539
- **WALSH, D. M. ; D. J. SELKOE.** Oligomers on the brain: the emerging role of soluble protein aggregates in neurodegeneration. *Protein Pept Lett,* 2004, vol. 11, 213-228
- **WALSH, D. M. ; D. J. SELKOE.** Aβ oligomers - a decade of discovery. *J Neurochem,* 2007, vol. 101, 1172-1184
- **WANG. J. ; D. W. DICKSON ; J. Q. TROJANOWSKI ; V. M. LEE.** The levels of soluble versus insoluble brain Abeta distinguish Alzheimer's disease from normal and pathologic aging. *Exp Neurol,* 1999, vol. 158, 328-337
- The pharmacological treatment of Alzheimer's disease with cholinesterase inhibitors and memantine. **WILCOCK, G. K.** Pharmacological Mechanisms in Alzheimer's Therapeutics. Springer, 2008, 36-49
- **WOOD, J. D. ; T. P. BEAUJEUX ; P. J. SHAW.** Protein aggregation in motor neurone disorders. *Neuropathol Appl Neurobiol,* 2003, vol. 29, 529-545